# EUROPEAN PATENT SPECIFICATION

(11) **EP 3 930 478 B1**
(45) Date of publication and mention of the grant of the patent: **29.01.2025**
(21) Application number: 20718000.1
(22) Date of filing: 25.02.2020
(51) Int. Cl.: A23L 2/02, A23L 5/20

(54) **STABLE PROTEIN FORMULATIONS**
STABILE PROTEINFORMULIERUNGEN
FORMULATIONS STABLES DE PROTÉINE

(30) Priority: 26.02.2019 US 201962810891 P
(43) Date of publication of application: 05.01.2022
(73) Proprietor: Amano Enzyme USA Co., Ltd., Elgin, IL 60124 (US); Amano Enzyme Inc., Nagoya-shi, Aichi 460-8630 (JP)
(72) Inventor: LIOUTAS, Theodore S., Elgin, Illinois 60124 (US); OKUDA, Keita, Elgin, Illinois 60124 (US)
(74) Representative: Müller-Boré & Partner Patentanwälte PartG mbB
(86) International application number: PCT/US2020/019646
(87) International publication number: WO 2020/176469

(56) References cited:
- EP-A1- 1 338 210
- EP-A1- 2 351 837
- EP-A1- 3 130 671
- WO-A1-2004/032648
- WO-A1-2017/009100
- WO-A2-2008/138900
- CN-A- 107 325 977
- SCHEUPLEIN ET AL: "Studies on the non-pathogenicity of Chryseobacterium proteolyticum and on the safety of the enzyme: Protein-glutaminase", REGULATORY TOXICOLOGY AND PHARMACOLOGY, ACADEMIC PRESS,NEW YORK, NY, US, vol. 49, no. 2, 30 October 2007 (2007-10-30), pages 79 - 89, XP022322193, ISSN: 0273-2300, DOI: 10.1016/J.YRTPH.2007.06.001

## Description

### FIELD

Described herein are stable protein solutions having a pH of 7 or lower that are stable against precipitation of the protein, as well as methods of making such stable protein solutions, and their use in beverages or beverages additives.

### BACKGROUND

There is growing demand for protein-rich food products and protein-rich vegetarian and vegan products. In particular, there is growing demand for protein-rich beverages and beverages additives comprising plant proteins and other non-animal proteins. However, formulating such proteins in solutions, particularly in solutions having pH 7 or lower as is common for beverages, is difficult due to the limited solubility of such proteins at pH 7 and lower. Thus, there is a need for protein solutions that are stable against precipitation of the protein at pH 7 and lower.

### SUMMARY

Provided herein are stable protein solutions, comprising (i) a protein; (ii) a stabilizer; and (iii) a protein deamidating enzyme, wherein the solution has a pH of from about 3.5 to about 7.0 and is stable against precipitation of the protein. The solution comprises (i) about 0.1% to about 30% w/v of the protein, based on the volume of the solution; (ii) about 0.001% to about 5% w/v of the stabilizer, based on the volume of the solution; and (iii) about 0.5 U to about 50 U of protein deamidating enzyme activity or about 0.1% to about 10% w/w of the protein deamidating enzyme, based on the weight of the protein in the solution. In some embodiments, the solution comprises (i) about 0.1% to about 30% w/v of the protein, based on the volume of the solution; (ii) about 0.001% to about 1% w/v of the stabilizer, based on the volume of the solution; and (iii) about 5 U to about 50 U of protein deamidating enzyme activity or about 1% to about 10% w/w of the protein deamidating enzyme, based on the weight of the protein in the solution. In some embodiments, the solution comprises about 5% to about 15% w/v of the protein, based on the volume of the solution. In some embodiments, the solution comprises about 0.02% to about 0.5% w/v of the stabilizer, based on the volume of the solution. In some embodiments, the solution comprises from about 1% w/w to about 5% w/w of the protein deamidating enzyme, based on the weight of the protein in the solution.

In some embodiments, the protein comprises one or more selected from a plant protein (such as soy, pea, lentil, chick pea, legume, hemp, rice, nut, wheat, and gluten proteins, including peanut protein and almond protein), a dairy protein (such as whey protein), and an insect protein (such as one or more of cricket, mole cricket, silk worm, sago worm, grasshopper, scorpion, diving beetle, waterbug, earth worm, mealworm, and spider proteins).

In some embodiments, the stabilizer comprises one or more of a gum, a polysaccharide, and a collagen, such as one or more of xanthan gum, gellan gum, carrageenan gum, cassia gum, locust bean gum, tara gum, psyllium seed gum, gelatin, tamarind seed gum, gum arabic, alginate, propylene glycol alginates, pectin, galactomannan (guar gum), pullulan, methylcellulose (MC), carboxymethylcellulose (CMC), and derivatives or combinations of any thereof.

In some embodiments, the protein deamidating enzyme deamidates amido groups of asparagine and/or glutamine residues of the protein, *e.g.*, is a protein glutaminase deamidating enzyme or a protein asparaginase deamidating enzyme. In some embodiments, the protein deamidating enzyme is produced by bacteria selected from *Chryseobacterium, Flavobacterium, Enpedobacter, Sphingobacterium, Aureobacterium, Myroides, Cytophagales, Actinomycetes,* and *Flavobacteriaceae.* In some embodiments, the protein deamidating enzyme is produced by a *Penicillium* microorganism. In some embodiments, the protein deamidating enzyme is Protein Glutaminase Amano 500 (PGA 500), which is a protein glutaminase deamidating enzyme. In some embodiments, the protein deamidating enzyme comprises the amino acid sequence of SEQ ID NO:1 (which is a protein glutaminase deamidating enzyme), or a sequence having at least 75%, at least 80%, at least 85%, at least 90%, at least 95%, at least 97%, at least 98%, or at least 99% identity thereto and having protein deamidating enzyme activity. In some embodiments, the protein deamidating enzyme comprises a variant amino acid sequence of SEQ ID NO:1, having one or more substitution or deletions at amino acid residues 35, 38-43, 45, 46, 49, 79-84, 103-106, 117, 142, 143, 146, 166, or 185 of SEQ ID NO:1. In some embodiments, the protein deamidating enzyme comprises a variant amino acid sequence of SEQ ID NO:1 that is at least 75%, at least 80%, at least 85%, at least 90%, at least 95%, at least 97%, at least 98%, or at least 99% identical thereto, having one or more substitution or deletions at amino acid residues 35, 38-43, 45, 46, 49, 79-84, 103-106, 117, 142, 143, 146, 166, or 185 of SEQ ID NO:1, and having protein deamidating enzyme activity.

In some embodiments, the solution has a pH of from about 4.0 to about 7.0 or from about 4.0 to about 5.0.

In some embodiments, the solution is stable against visible precipitation of the protein after storage at 4°C for a period of time selected from 7 days, 14 days, 21 days, 1 month, 2 months, and 6 months, including 3 months, 4 months, 5 months, 6 months, 7 months, 8 months, 9 months, 10 months, and 12 months.

In some embodiments, the solution is formulated as a beverage or beverage additive for human or animal consumption.

Also provided are beverages or beverage additives for human or animal consumption, comprising a stable protein solution as described herein. In some embodiments, the beverage or beverage additive is selected from a nutritional beverage, a sports drink, a functional protein drink, a dairy drink, a dairy smoothie, a fruit drink, a fruit smoothie, a coffee drink, a tea drink, a plant milk, a dairy creamer, and a non-dairy creamer. In some embodiments, the beverage or beverage additive comprises one or more acidic or fruit juices or acidic or fruit juice concentrates. In some embodiments, the beverage or beverage additive comprises one or more vegetable juices or vegetable concentrates. In some embodiments, the beverage or beverage additive comprises one or more acidic fruit, or vegetable juices or acidic fruit, or vegetable juice concentrates.

Also provided are methods of making a stable protein solution as described herein, or a beverage or beverage additive as described herein, comprising (a) adding protein deamidating enzyme to a solution comprising the protein and the stabilizer to obtain a mixture; (b) incubating the mixture; and (c) acidifying the mixture to obtain a solution with a pH of from about 3.5 to about 7.0. In some embodiments, the solution is prepared by mixing (i) a solution comprising the protein and (ii) a solution comprising the stabilizer. In some embodiments, the incubating is conducted until the enzyme reaction reaches a desired level of completion, optionally as determined by the concentration of free ammonium ions in the solution. In some embodiments, the incubating is at a temperature of from about 30°C to about 70°C and for a period of from about 0.5 hours to about 48 hours, optionally with agitation, optionally at a pH of from about 3.0 to about 8.0. In some embodiments, the incubating is at a temperature of from about 40°C to about 60°C and for a period of from about 3 hours to about 24 hours, optionally with agitation, optionally at a pH of from about 5.0 to about 8.0. In some embodiments, the acidifying comprises adding an acidic juice or juice concentrate. In some embodiments, the protein deamidating enzyme is Protein Glutaminase Amano 500 (PGA 500) and/or has the amino acid sequence of SEQ ID NO:1 or a variant thereof as described herein, and the incubating is at 50°C for 3 hours.

In some embodiments, the process further comprises subjecting the solution to a heat treatment of about 85°C for about 10 minutes. In some embodiments, the process further comprises subjecting the solution to one or more treatments selected from homogenization, pasteurization, and sterilization. In some embodiments, the homogenization is performed at a pressure of from about 2,000 psi to about 20,000 psi, including from about 2,000 psi to about 2,500 psi. In some embodiments, the pasteurization is performed using High Temperature Short Time (HTST) pasteurization at about 100°C for about 10 seconds to about 20 seconds, Ultra High Temperature (UHT) pasteurization at about 120°C for about 1 second to about 3 seconds, or Low Temperature Long Time (LTLT) pasteurization at from about 75°C to about 85°C for about 10 minutes to about 20 minutes. In some embodiments, the sterilization is performed using high pressure (hyperbaric) sterilization.

### BRIEF DESCRIPTION OF THE DRAWINGS

FIG. 1 shows the absorbance of pea protein formulations with (i) protein glutaminase deamidating enzyme and gum; (ii) protein glutaminase deamidating enzyme; (iii) gum; and (iv) without protein glutaminase deamidating enzyme and without gum by pH.
FIG. 2 shows the absorbance of soy protein formulations with (i) protein glutaminase deamidating enzyme and gum; (ii) protein glutaminase deamidating enzyme; (iii) gum; and (iv) without protein glutaminase deamidating enzyme and without gum by pH.
FIG. 3 shows the absorbance of hemp protein formulations with (i) protein glutaminase deamidating enzyme and gum; (ii) protein glutaminase deamidating enzyme; (iii) gum; and (iv) without protein glutaminase deamidating enzyme and without gum by pH.
FIG. 4 shows the absorbance of peanut protein formulations with (i) protein glutaminase deamidating enzyme and gum; (ii) protein glutaminase deamidating enzyme; (iii) gum; and (iv) without protein glutaminase deamidating enzyme and without gum by pH.
FIG. 5 shows the absorbance of cricket protein formulations with (i) protein glutaminase deamidating enzyme and gum; (ii) protein glutaminase deamidating enzyme; (iii) gum; and (iv) without protein glutaminase deamidating enzyme and without gum by pH.
FIG. 6 shows the absorbance of homogenized pea protein formulations with (i) protein glutaminase deamidating enzyme and gum; and (ii) gum alone (without protein glutaminase deamidating enzyme).
FIG. 7 shows the absorbance of homogenized soy protein formulations containing juice concentrate with (i) protein glutaminase deamidating enzyme and gum; and (ii) gum alone (without protein glutaminase deamidating enzyme).
FIG. 8 shows the absorbance of homogenized peanut protein formulations containing juice concentrate with (i) protein glutaminase deamidating enzyme and gum; and (ii) gum alone (without protein glutaminase deamidating enzyme).

### DETAILED DESCRIPTION

### Definitions

Technical and scientific terms used herein have the meanings commonly understood by one of ordinary skill in the art to which the present disclosure pertains, unless otherwise defined. Reference is made herein to various methodologies known to those of ordinary skill in the art. Suitable materials and/or methods known to those of ordinary skill in the art can be utilized in carrying out the present disclosure. However, specific materials and methods are described for illustration only. Materials, reagents and the like to which reference is made in the following description and examples are obtainable from commercial sources, unless otherwise noted.

As used herein, the singular forms "a," "an," and "the" designate both the singular and the plural, unless expressly stated to designate the singular only.

As used herein, the term "about" means that the number or range is not limited to the exact number or range set forth, but encompass values around the recited number or range as will be understood by persons of ordinary skill in the art depending on the context in which the number or range is used. Unless otherwise apparent from the context or convention in the art, "about" means up to plus or minus 10% of the particular term.

Described herein are stable protein solutions comprising a protein, a stabilizer, and a protein deamidating enzyme, where the protein solutions have a pH of from about 3.5 to about 7.0 and are stable against precipitation of the protein. Also described herein are beverages and beverages additives comprising such solutions. Also described herein are methods of making such stable protein solutions, and methods of making beverages or beverage additives comprising them.

As used herein, "stable against precipitation of the protein" means that there is no visible precipitation of protein. In some embodiments, no visible precipitation is confirmed by assessing absorbance at about 280 nm, wherein increased absorbance is correlated with solubilized protein and lack of precipitation. With solutions having the concentrations of protein described herein (without precipitation), typical absorbance at about 280 nm is in the range of from about 8 to 50 mg protein/mL.

The stable protein solutions described herein address the problem of formulating proteins in solutions having pH 7 or lower, which is a common pH for beverages and beverage additives. For example, many beverages, including functional beverages and sports beverages, contain juices from fruits and/or vegetables or juices flavors and have a pH of 7 or lower, such as a pH of about 7 to about 3.5. When proteins are formulated in such beverages, they have a tendency to precipitate out of solution, resulting in sedimentation. Without being bound by theory, this precipitation is believed to be due to the pH of the beverage being close to the isoelectric point of the protein, which causes destabilization of the protein and its precipitation and sedimentation. In addition to being unacceptable to consumers, the precipitation of the proteins limits flavor-masking options and other formulation options. The stability of solutions described herein at acidic pH permit formulating a protein solution with an acidic juice, such as a fruit juice. As illustrated in the examples below, solutions as described herein are stable against precipitation even at an acidic pH. Thus the solutions described herein permit formulating a protein solution in or with an acidic juice, such as fruit juice, such as to provide a protein-containing fruit juice-based or fruit- or fruit juice-flavored beverage or beverage additive.

The stable protein solutions described herein use a unique combination of a protein deamidating enzyme and a stabilizer to address this problem. While protease enzymes have been used previously, their use is limited by the formation of compounds with unwanted flavors that result from enzyme degradation of the substrate proteins. While certain gum stabilizers and emulsifiers have been used previously, they are only effective at high concentrations (e.g., 2-5% w/v) that exert other unwanted effects such as coagulation, stratification and even precipitation. Further, the use of stabilizers at these high concentrations leads to final products with high viscosities that are undesirable to consumers. In contrast, solutions as described herein have acceptable viscosity properties for use in or as beverages and beverage additives, such as viscosities ranging from about 10 to about 250 mPa·s. (For reference, milk has a viscosity of about 2-3 mPa·s, most vegetable oils have a viscosity of about 40-50 mPa·s, and a chocolate sauce may have a viscosity of 280 mPa·s.)

Without being bound by theory, the protein deamidating enzymes described herein are believed to deamidate amino acid residues, such as glutamine and/or asparagine residues, in the protein, thereby increasing the negative charge of the protein, decreasing the isoelectric point of the protein, and increasing its solubility at acidic pH values. As a result, protein solubility at an acidic pH is improved. Also without being bound by theory, certain of the protein deamidating enzymes described herein increase protein solubility without producing unwanted flavor compounds by deamidating the protein without breaking peptide bonds, such as by deamidating the amido groups of amino acid residues in the protein, including converting glutamine residues in the protein into glutamic acid and/or converting asparagine residues in the protein into aspartic acid.

Although enzyme treatment alone can increase protein solubility to some extent, further formulation approaches are needed to provide solutions that are stable against precipitation of the protein at pH 7 and lower over extended periods of time, such as over storage conditions typical for consumer beverage and beverage additive products. Thus, the solutions described herein include stabilizers that further promote the stability of protein solutions, and permit the preparation of solutions having a pH from about 3.5 to about 7 that are stable against precipitation of the protein under refrigerated conditions for extended periods of time, such as a period of time of 7 days, 14 days, 21 days, 1 month, 2 months, 3 months, 4 months, 5 months, 6 months, 7 months, 8 months, 9 months, 10 months, and 12 months, such as 4 months or 8 months , under storage at 4°C. Unlike previously described formulations, the solutions described herein only require relatively small amounts of stabilizers, such that the use of stabilizers as described herein does not undermine the physicochemical properties of the solution or lead to effects that are unacceptable to consumers, such as coagulation, stratification, precipitation, or high viscosity.

Solutions described herein may be subject to homogenization, and exhibit stability against protein precipitation after homogenization, as illustrated in the examples below. Thus, even though the homogenization process may cause changes in protein-protein interactions, protein formulated in a solution as described herein may remain in solution even after homogenization.

As noted above, in accordance with specific embodiments, there are provided stable protein solutions comprising (i) a protein; (ii) a stabilizer; and (iii) a protein deamidating enzyme, wherein the solution has a pH of from about 3.5 to about 7.0 and is stable against precipitation of the protein. Specific aspects and specific embodiments are discussed in more detail below.

### Protein

The proteins that can be formulated as described herein are not limited, but embodiments of interest include proteins suitable for human or animal consumption, including animal, plant, dairy, and insect proteins suitable for human or animal consumption. In some embodiments, a solution as described herein comprises one or more proteins selected from a plant protein, a dairy protein, and an insect protein.

Examples of suitable plant proteins include, but are not limited to, soy, pea, lentil, chick pea, legume, hemp, rice, nut, wheat, and gluten proteins. In some embodiments, the plant protein is selected from one or more of soy, pea, lentil, chick pea, legume, hemp, rice, nut, wheat, and gluten proteins. In some embodiments, the nut is peanut, almond, or hazelnut. In some embodiments, the protein comprises pea protein. In some embodiments, the protein comprises soy protein. In some embodiments, the protein comprises peanut protein. In some embodiments, the protein comprises hemp protein.

Examples of suitable dairy proteins include, but are not limited to, whey protein. In some embodiments, the protein comprises whey protein.

Examples of suitable insect proteins, include but are not limited to, cricket, mole cricket, silk worm, sago worm, grasshopper, scorpion, diving beetle, waterbug, earth worm, mealworm, and spider proteins. In some embodiments, the protein comprises an insect protein selected from one or more of cricket, mole cricket, silk worm, sago worm, grasshopper, scorpion, diving beetle, waterbug, earth worm, mealworm, and spider proteins. In some embodiments, the protein comprises cricket protein.

### Stabilizer

As noted above, the solutions described herein include a stabilizer. The stabilizer comprises one or more of xanthan gum, gellan gum, carrageenan gum, cassia gum, locust bean gum, tara gum, psyllium seed gum, gelatin, tamarind seed gum, gum arabic, alginate, propylene glycol alginates, pectin, galactomannan (guar gum), pullulan, carboxymethylcellulose (CMC), methylcellulose (MC), and derivatives or combinations of any thereof. In specific embodiments, the stabilizer is selected from xanthan gum, gellan gum, carrageenan gum, tara gum, pectin, alginate, and CMC. In some embodiments, the stabilizer comprises gellan gum. In some embodiments, the stabilizer comprises carrageenan gum. In some embodiments, the stabilizer comprises pectin gum. In some embodiments, the stabilizer comprises xanthan gum. As illustrated in the examples below different stabilizers may be more effective at different pH ranges or different pH values. Thus, the choice of stabilizer may be guided in some respects by the pH of the final product.

### Protein Deamidating Enzyme

As noted above, the solutions described herein include a protein deamidating enzyme. As used herein, a "protein deamidating enzyme" is an enzyme that deamidates amido groups of amino acid residues of the protein. In some embodiments, the protein deamidating enzyme deamidates amido groups of asparagine and/or glutamine residues of the protein. In some embodiments, the protein deamidating enzyme deamidates amido groups of glutamine residues of the protein. In some embodiments, the protein deamidating enzyme deamidates amido groups of asparagine residues of the protein. Examples of suitable protein deamidating enzymes include those described in U.S. Patent No. 6,756,221, U.S. Patent No. 6,251,651, U.S. Patent No. 7,462,477, and U.S. Patent No.8,735,131, and in particular for the protein deamidating enzymes disclosed therein WO2008/138900 addresses the reduction or prevention of the formation of precipitations or aggregates of casein in acidified milk drinks, whereby the liquid separates over time during storage, and whey collects at the surface (syneresis). It addresses solubility of protein at acidic pH, in particular in beverages, and substitutes stabilizers CMC or pectin by deamidation of the protein. It does not disclose the presence of small amounts of stabiliser in addition to the deamidation.

In some embodiments, the protein deamidating enzyme is produced by bacteria selected from *Chryseobacterium, Flavobacterium, Enpedobacter, Sphingobacterium, Aureobacterium, Myroides, Cytophagales, Actinomycetes,* and *Flavobacteriaceae,* or by a *Penicillium* microorganism. In some embodiments, the protein deamidating enzyme is produced by bacteria from *Chryseobacterium.* In some embodiments, the protein deamidating enzyme is produced by bacteria from *Flavobacterium.* In some embodiments, the protein deamidating enzyme is produced by bacteria from *Enpedobacter.* In some embodiments, the protein deamidating enzyme is produced by bacteria from *Sphingobacterium.* In some embodiments, the protein deamidating enzyme is produced by bacteria from *Aureobacterium.* In some embodiments, the protein deamidating enzyme is produced by bacteria from *Myroides.* In some embodiments, the protein deamidating enzyme is produced by bacteria from *Cytophagales.* In some embodiments, the protein deamidating enzyme is produced by bacteria from *Actinomycetes.* In some embodiments, the protein deamidating enzyme is produced by bacteria from *Flavobacteriaceae.*

In some embodiments, the protein deamidating enzyme is the protein glutaminase deamidating enzyme Protein Glutaminase Amano 500 (PGA 500), available commercially from Amano Enzyme.

In some embodiments, the protein deamidating enzyme has or comprises the amino acid sequence of SEQ ID NO:1 (which is a protein glutaminase deamidating enzyme), or a sequence at least 75%, at least 80%, at least 85%, at least 90%, at least 95%, at least 97%, at least 98%, or at least 99% identical thereto and having protein deamidating enzyme activity. The degree of the protein deamidating enzyme activity is not particularly limited as long as the function of a protein deamidating enzyme can be exhibited, but is preferably equivalent to or higher than that of the enzyme having an amino acid sequence of SEQ ID NO: 1. In some embodiments, the protein deamidating enzyme comprises a variant amino acid sequence of SEQ ID NO:1, having one or more substitution or deletions at amino acid residues 35, 38-43, 45, 46, 49, 79-84, 103-106, 117, 142, 143, 146, 166, or 185 of SEQ ID NO:1. In some embodiments, the protein deamidating enzyme comprises a variant amino acid sequence of SEQ ID NO:1 that is at least 75%, at least 80%, at least 85%, at least 90%, at least 95%, at least 97%, at least 98%, or at least 99% identical thereto, having one or more substitution or deletions at amino acid residues 35, 38-43, 45, 46, 49, 79-84, 103-106, 117, 142, 143, 146, 166, or 185 of SEQ ID NO:1, and having protein deamidating enzyme activity. In some embodiments, the protein deamidating enzyme has or comprises a variant amino acid sequence of sequence of SEQ ID NO:1, having one or more substitution or deletions at amino acid residues 39, 40, 41, 43, 79-82, 142, 143, 146, 166, or 185 of SEQ ID NO:1, such as one or more substitution or deletions at amino acid residues 35, 38, 40-43, 45, 46, 49, 80-84, 103-106, or 117 of SEQ ID NO:1, as described in U.S. Patent No. 8,735,131. In some embodiments, the protein deamidating enzyme has or comprises a variant amino acid sequence of SEQ ID NO:1, having one or more substitution or deletions at amino acid residues 82 or 84 of SEQ ID NO:1 as described in U.S. Patent No. 8,735,131. In some embodiments, the protein deamidating enzyme has or comprises a variant amino acid sequence of SEQ ID NO:1, such as a substitution at amino acid residue 82 of SEQ ID NO:1, such as a serine substitution at amino acid residue 82 of SEQ ID NO:1 and/or a substitution at amino acid residue 84 of SEQ ID NO:1, such as an aspartic acid substitution at amino acid residue 84 of SEQ ID NO:1, as described in U.S. Patent No. 8,735,131.

### Stable Protein Solutions

As noted above, the stable protein solutions described herein comprise:
(i) about 0.1% to about 30% w/v of the protein, based on the volume of the solution;
(ii) about 0.001% to about 5%, including about 0.001% to about 1%, w/v of the stabilizer, based on the volume of the solution; and
(iii) about 0.5 to about 50 U of protein deamidating enzyme activity or about 0.1% to about 10%, including about 1% to about 10%, w/w of the protein deamidating enzyme, based on the weight of the protein in the solution, where protein deamidating enzyme activity may be determined in accordance with the assay of Example 16 below.

Thus, the solution includes about 0.1% to about 30% w/v of the protein, based on the volume of the solution (*e.g.*, the final volume of the solution), In some embodiments, the solution comprises from about 1% to about 15% w/v of the protein, based on the volume of the solution. In some embodiments, the solution comprises from about 5% to about 15% w/v of the protein, based on the volume of the solution. In some embodiments, the solution comprises about 1%, about 2%, about 3%, about 4%, about 5%, about 6%, about 7%, about 8%, about 9%, about 10%, about 11%, about 12%, about 13%, about 14%, about 15%, about 20%, about 25%, or about 30% w/v of the protein, based on the volume of the solution, including 1%, 2%, 3%, 4%, 5%, 6%, 7%, 8%, 9%, 10%, 11%, 12%, 13%, 14%, 15%, 20%, 25%, or 30% w/v of the protein, based on the volume of the solution.

The solution includes about 0.001% to about 5% w/v of the stabilizer, based on the volume of the solution, including from about 0.001% to about 1.5%, about 0.001% to about 2%, about 0.001% to about 3%, and about 0.001% to about 4% w/v of the stabilizer, based on the volume of the solution. In some embodiments, the solution includes about 0.001% to about 1% w/v of the stabilizer, based on the volume of the solution, such as from about 0.01% to about 1%, about 0.01% to about 0.5%, and about 0.02% to about 0.5% w/v of the stabilizer, based on the volume of the solution. In some embodiments, the solution comprises about 0.02%, about 0.03%, about 0.04%, about 0.05%, about 0.06%, about 0.07%, about 0.08%, about 0.09%, about 0.1%, about 0.2%, about 0.3%, about 0.4%, about 0.5% w/v of the stabilizer, based on the volume of the solution, including 0.02%, 0.03%, 0.04%, 0.05%, 0.06%, 0.07%, 0.08%, 0.09%, 0.1%, 0.15%, 0.2%, 0.25%, 0.3%, 0.35%, 0.4%, 0.45%, or 0.5% w/v of the stabilizer. In some embodiments, the solution comprises about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5%, about 0.6%, about 0.7%, about 0.8%, about 0.9%, about 1.0%, about 1.5%, about 2%, about 3%, about 4% or about 5% w/v of the stabilizer, based on the volume of the solution, including 0.02%, 0.03%, 0.04%, 0.05%, 0.06%, 0.07%, 0.08%, 0.09%, 0.1%, 0.15%, 0.2%, 0.25%, 0.3%, 0.35%, 0.4%, 0.45%, 0.5%, 0.6%, 0.7%, 0.8%, 0.9%, 1.0%, 1.5%, 2%, 3%, 4%, and 5% w/v of the stabilizer. In some embodiments, the relatively low amount of stabilizer used yields a solution that has a viscosity that is acceptable to consumers for beverages and beverage additives, such as a viscosity of from about 10 to about 250 mPa·s.

In some embodiments, the solution includes from about 0.1% w/w to about 10% w/w of the protein deamidating enzyme, based on the weight of the protein in the solution, including about 0.1% w/w to about 1.0% w/w, about 0.5% to about 1.0% w/w, about 0.1% w/w, about 0.2% w/w, about 0.3% w/w, about 0.4% w/w, about 0.5% w/w, or about 0.6% w/w, about 0.7% w/w, about 0.8% w/w, or about 0.9% w/w, based on the weight of the protein in the solution. In some embodiments, the solution includes from about 1% w/w to about 10% w/w of the protein deamidating enzyme, based on the weight of the protein in the solution. In some embodiments, the solution comprises from about 1% w/w to about 5% w/w of the protein deamidating enzyme, such as about 1% w/w, about 2% w/w, about 3% w/w, about 4% w/w, or about 5% w/w of the protein deamidating enzyme, based on the weight of the protein in the solution, including 1%, 2%, 3%, 4%, 5%, 6%, 7%, 8%, 9%, or 10%.

In some embodiments, the solution includes from about 0.5 U to about 50 U of protein deamidating enzyme activity, including about 0.5 to about 5.0 U, about 2.5 to about 5.0 U, about 0.5 U, about 1.0 U, about 2.0 U, about 2.5 U, about 3.0 U, about 4.0 U or about 5.0 U. In some embodiments, the solution includes from about 5 U to about 50 U of protein deamidating enzyme activity. In some embodiments, the solution comprises from about 5 U to about 25 U of protein deamidating enzyme activity, such as about 5 U, about 10 U, about 15 U, about 20 U, or about 25 U of protein deamidating enzyme activity, including 5 U, 10 U, 15U, 20 U, 25 U, 30 U, 35 U, 40 U, 45 U, or 50 U. The protein deamidating enzyme activity may be determined as described below in Example 16.

The solution (or beverage or beverage additive comprising a solution as described herein) has a pH of from about 3.5 to about 7, including from 3.5 to 7, such as from about 3.5 to about 5.5, including from 3.5 to 5.5. In some embodiments, the solution (or beverage or beverage additive comprising a solution as described herein) has a pH of from about 4.0 to about 5.0, including from 4.0 to 5.0. In some embodiments, the solution (or beverage or beverage additive comprising a solution as described herein) has a pH of from about 4.0 to about 7.0, including from 4.0 to 7.0, such as a pH of about 3.5, about 4.0, about 4.5, about 5.0, about 5.5, about 6.0, about 6.5, or about 7.0, including 3.5, 4.0, 4.5, 5.0, 5.5, 6.0, 6.5, or 7.0.

In some embodiments, the viscosity of the solution (or beverage or beverage additive comprising a solution as described herein) is from about 10 to about 250 mPa·s, including from 10 to 250 mPa·s. In some embodiments, the viscosity of the solution (or beverage or beverage additive comprising a solution as described herein) is about 10, about 20, about 30, about 40, about 50, about 60, about 70, about 80, about 90, about 100, about 110, about 120, about 130 about 140, about 150, about 160, about 170, about 180, about 190, about 200, about 210, about 220, about 230, about 240, or about 250 mPa·s, including 10, 20, 30, 40, 50, 60, 70, 80, 90, 100, 110, 120, 130 140, 150, 160, 170, 180, 190, 200, 210, 220, 230, 240, or 250 mPa·s. Viscosity can be measured using an AMETEK BROOKFIELD viscometer using spindle S61 at room temperature (~20°C).

In some embodiments, the solution (or beverage or beverage additive comprising a solution as described herein) is stable against visible precipitation of the protein after storage at 4°C for a period of time selected from 7 days, 14 days, 21 days, 1 month, 2 months, and 6 months. In some embodiments, the solution is stable against visible precipitation of the protein after storage at 4°C for a period of time selected from 7 days, 14 days, 21 days, 1 month, 2 months, 3 months, 4 months, 5 months, 6 months, 7 months, 8 months, 9 months, 10 months, and 12 months, such as for 4 months, or for 8 months. In some embodiments, the solution (or beverage or beverage additive comprising a solution as described herein) is stable against visible precipitation of the protein after storage at 4°C for 7 days. In some embodiments, the solution (or beverage or beverage additive comprising a solution as described herein) is stable against visible precipitation of the protein after storage at 4°C for 14 days. In some embodiments, the solution (or beverage or beverage additive comprising a solution as described herein) is stable against visible precipitation of the protein after storage at 4°C for 21 days. In some embodiments, the solution (or beverage or beverage additive comprising a solution as described herein) is stable against visible precipitation of the protein after storage at 4°C for 1 month. In some embodiments, the solution (or beverage or beverage additive comprising a solution as described herein) is stable against visible precipitation of the protein after storage at 4°C for 2 months. In some embodiments, the solution (or beverage or beverage additive comprising a solution as described herein) is stable against visible precipitation of the protein after storage at 4°C for 4 months. In some embodiments, the solution (or beverage or beverage additive comprising a solution as described herein) is stable against visible precipitation of the protein after storage at 4°C for 6 months. In some embodiments, the solution (or beverage or beverage additive comprising a solution as described herein) is stable against visible precipitation of the protein after storage at 4°C for 8 months. As noted above and illustrated in the examples below, stability against precipitation also can be assessed by measuring absorbance at 280 nm, with higher absorbance being correlated with solubilized protein and, hence, reduced or no precipitation.

### Beverage or Beverage Additive

The proteins solutions described herein may be formulated as beverages or beverage additives for human or animal consumption, or may be used to prepare beverages or beverage additives for human or animal consumption. Examples of beverages or beverage additives include, but are not limited to, nutritional beverages, sports drinks, functional protein drinks, dairy drinks, dairy smoothies, fruit drinks, fruit smoothies, coffee drinks, tea drinks, plant milks, dairy creamers, and non-dairy creamers. In some embodiments, the beverage or beverage additive is selected from a nutritional beverage, a sports drink, a functional protein drink, a dairy drink, a dairy smoothie, a fruit drink, a fruit smoothie, a coffee drink, a tea drink, a plant milk, a dairy creamer, and a non-dairy creamer. The beverage or beverage additive may further comprise one or more fruit juices, vitamins, and flavoring agents.

As noted above, in some embodiments, the beverage or beverage additive comprises one or more acidic juices, such as one or more fruit or vegetable juices, including mixtures of fruit and vegetable juices, including an acidic fruit juice and/or an acidic vegetable juice. Examples of such juices include apple juice, cherry juice, cranberry juice, grape juice, pineapple juice, pomegranate juice, grapefruit juice, guava juice, honeydew juice, lime juice, lemon juice, blackberry juice, orange juice, pineapple juice, raspberry juice, banana puree, apricot juice, peach juice, acai puree, acai juice, kiwifruit juice, sugarcane juice, strawberry juice, watermelon juice, passion fruit juice, celery juice, carrot juice, potato juice, beet juice, parsley juice, tomato juice, watercress juice and turnip juice. As noted above, the present disclosure of protein solutions that are stable against precipitation at acidic pH permits formulating a protein solution in or with a fruit and/or vegetable juice, such as to provide a protein-containing fruit and/or vegetable juice-based or fruit- and/or vegetable or fruit and/or vegetable juice- flavored beverage or beverage additive.

### Methods of Preparation

Also described herein are methods of making a stable protein solution as described herein, and methods of making beverages and beverage additives. The methods comprises (a) adding protein deamidating enzyme to a solution comprising the protein and the stabilizer to obtain a mixture; (b) incubating the mixture; and (c) acidifying the mixture to obtain a solution with a pH of from about 3.5 to about 7.0. In some embodiments, the solution is prepared by mixing (i) a solution comprising the protein and (ii) a solution comprising the stabilizer. The methods comprises providing a mixture comprising the protein and stabilizer, and adding the protein deamidating enzyme to the mixture and incubating the mixture. In some embodiments, the incubating is conducted until the enzyme reaction reaches a desired level of completion, optionally as determined by the concentration of free ammonium ions in the solution. The methods comprise mixing a solution comprising the protein with a solution comprising the stabilizer to provide a mixture comprising the protein and stabilizer, and adding the protein deamidating enzyme to the mixture and incubating the mixture. The mixing is completed before the enzyme is added.

The methods also comprises adjusting the pH of the solution to a pH of from about 3.5 to about 7.0, such as by acidifying the solution to a pH of from about 3.5 to about 7.0. In some embodiments, acidifying the solution comprises adding an acidic juice or juice concentrate, such as an acidic fruit juice or acidic fruit juice concentrate and/or an acidic vegetable juice or acidic vegetable juice concentrate. In some embodiments, the solution is acidified by more than one acidifying agent, such as, for example, an acidic additive and an acidic juice or juice concentrate. In some embodiments, the acidifying agent is added for other purposes, such as for flavoring the solution or enhancing the nutritional or nutraceutical content thereof, and the acidic pH results from the amount of acidifying agent added for that purpose.

The incubating conditions can be any incubating conditions suitable for the specific protein deamidating enzyme(s) used, such as any temperature and pH at which the enzyme is active and any time period required to achieve the desired level of deamidation. In some embodiments, the progress of the deamidation reaction is monitored, such as by measuring the concentration of free ammonium ions in the solution. For example, when the concentration of free ammonium ions in the solution reaches a specific level, the reaction may be considered to be complete. For solutions having the amounts of protein described herein, the reaction may be considered to be complete when the concentration of free ammonium ions in the solution reaches from about 0.002% to about 0.07% w/v, based on the volume of the solution, such as from 0.002% to 0.07% w/v, including about 0.002%, about 0.003%, about 0.004%, about 0.005%, about 0.006%, about 0.007%, about 0.008%, about 0.009%, about 0.01%, about 0.02%, about 0.03%, about 0.04%, about 0.05%, about 0.06%, or about 0.07% w/v, or 0.002%, 0.003%, 0.004%, 0.005%, 0.006%, 0.007%, 0.008%, 0.009%, 0.01%, 0.02%, 0.03%, 0.04%, 0.05%, 0.06%, or 0.07% w/v, based on the volume of the solution. The incubating conditions can include agitation. The agitation may be slow (*e.g.* about 150 to about 250 rpm) or fast (*e.g.* about 3,000 to about 5,000 rpm). In some embodiments, the agitation is performed with a shaking table with agitation in the range of from about 150 to about 250 rpm. In some embodiments, the agitation is performed with a shaking table with agitation in the range of from about 3,000 to about 5,000 rpm.

The incubating step may be at a temperature of from about 30°C to about 70°C, and for a period of time of from about 0.5 hours to about 48 hours, at pH of from about 3.0 to about 8.0. In general, the incubating will be at a temperature of from about 40°C to about 60°C, and for a period of time of from about 3 hours to about 24 hours, at pH of from about 5.0 to about 8.0. In some embodiments, the incubating is at a temperature of about 30°C, about 35°C, about 40°C, about 45°C, about 50°C, about 55°C, about 60°C, about 65°C, or about 70°C. In some embodiments, the incubating is for about 0.5 hour, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 48 hours. In some embodiments, the incubating is at a pH of about 3, about 3.5, about 4, about 4.5, about 5.0, about 5.5, about 6.0, about 6.5, about 7.0, about 7.5, or about 8.0.

In some embodiments, the protein deamidating enzyme is Protein Glutaminase Amano 500 (PGA 500) and the incubating is at 50°C for 3 hours at a pH of from about 5.0 to about 8.0. In some embodiments, the protein deamidating enzyme has or comprises the amino acid sequence of SEQ ID NO:1, and the incubating is at 50°C for 3 hours at a pH of from about 5.0 to about 8.0. In some embodiments, the protein deamidating enzyme is a variant of SEQ ID NO:1 as described herein, and the incubating is at 50°C for 3 hours at a pH of from about 5.0 to about 8.0.

The solution may be subject to one or more further processing steps, such one or more of the addition of one or more flavoring or nutritional ingredients, heat treatment, homogenization, filtration, pasteurization, and sterilization.

In some embodiments, the method further comprises subjecting the solution to a heat treatment, such as a heat treatment of from about 75°C to about 95°C for from about 5 minutes to about 20 minutes. In some embodiments, the heat treatment is conducted at about 75°C, about 80°C, about 85°C, about 90°C, or about 95°C, for about 5 minutes, 10 minutes, 15 minutes, or about 20 minutes. In some embodiments, the heat treatment is conducted at about 85°C for about 10 minutes.

In some embodiments, the process further comprises subjecting the solution to homogenization. In some embodiments, the homogenization is performed at a pressure of from about 2,000 psi to about 20,000 psi, such as from about 2,000 psi to about 2,500 psi. In some embodiments, the homogenization is performed at a pressure of from about 2,000 psi, about 5,000 psi, about 10,000 psi, about 15,000 psi, or about 20,000 psi. In some embodiments, the homogenization is performed at a pressure of about 2,000 psi, about 2,500 psi, about 3,000 psi, about 3,500 psi, about 4,000 psi, about 4,500 psi, or about 5,000 psi.

In some embodiments, the process further comprises subjecting the solution to pasteurization. In some embodiments, the pasteurization is performed using High Temperature Short Time (HTST) pasteurization, Ultra High Temperature (UHT) pasteurization, or Low Temperature Long Time (LTLT) pasteurization. In some embodiments, the pasteurization is performed using High Temperature Short Time (HTST) pasteurization. In some embodiments, the pasteurization is performed using High Temperature Short Time (HTST) pasteurization at from about 90°C to about 110°C for about 5 seconds to about 30 seconds. In some embodiments, the pasteurization is performed using High Temperature Short Time (HTST) pasteurization at about 100°C for about 10 seconds to about 20 seconds. In some embodiments, the pasteurization is performed using Ultra High Temperature (UHT) pasteurization. In some embodiments, the pasteurization is performed using Ultra High Temperature (UHT) pasteurization at from about 110°C to about 130°C for about 1 second to about 10 seconds. In some embodiments, the pasteurization is performed using Ultra High Temperature (UHT) pasteurization at about 120°C for about 1 second to about 3 seconds. In some embodiments, the pasteurization is performed using Low Temperature Long Time (LTLT) pasteurization. In some embodiments, the pasteurization is performed using Low Temperature Long Time (LTLT) pasteurization at from about 65°C to about 95°C for about 5 minutes to about 30 minutes. In some embodiments, the pasteurization is performed using Low Temperature Long Time (LTLT) pasteurization at from about 75°C to about 85°C for about 10 minutes to about 20 minutes.

In some embodiments, the process further comprises subjecting the solution to sterilization. In some embodiments, the sterilization is performed using high pressure (hyperbaric) sterilization.

Methods of making a beverage or beverage additive as described herein may comprise adding a stable protein solution as described herein to a beverage or beverage additive composition, or formulating a solution as described herein as a beverage or beverage additive. For example, a stable protein solution as described herein can be added to a pre-formulated nutritional beverage, sports drink, functional protein drink, dairy drink, dairy smoothie, fruit drink, fruit smoothie, coffee drink, tea drink, plant milk, dairy creamer, or non-dairy creamer, in an amount to provide the desired amount of protein in the beverage or beverage additive. Alternatively, a stable protein solution as described herein can be formulated as a nutritional beverage, a sports drink, a functional protein drink, a dairy drink, a dairy smoothie, a fruit drink, a fruit smoothie, a coffee drink, a tea drink, a plant milk, a dairy creamer, or a non-dairy creamer, e.g., comprising the other components of such a beverage and beverage additive and the desired amount of protein.

In some embodiments, the final solution, beverage, or beverage additive has a protein content of up to about 30% w/w, based on weight of protein in the solution, including about 30% w/w. In some embodiments, the final solution, beverage, or beverage additive has a protein content of from about 0.5 to about 30% w/w, based on weight of protein in the solution, including from 0.5 to 30% w/w, or from about 5 to about 25% w/w, or from about 10 to about 20% w/w. In some embodiments, the final solution, beverage, or beverage additive has a protein content of about 0.5%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, or about 30% w/w, based on the weight of protein in the solution.

In any embodiments, the beverage or beverage additive may further comprise one or more components typically present in such beverages or beverage additives, including one or more fruit or vegetable juices, vitamins, nutritional supplements, flavoring agents, coloring agents, and preservatives. In some embodiments, the beverage or beverage additive comprises one or more acidic juices or one or more fruit and vegetable juices, including one or more acidic fruit juices, including one or more selected from apple juice, cherry juice, cranberry juice, grape juice, pineapple juice, pomegranate juice, grapefruit juice, guava juice, honeydew juice, lime juice, lemon juice, blackberry juice, orange juice, pineapple juice, raspberry juice, banana puree, apricot juice, peach juice, acai puree, acai juice, kiwifruit juice, sugarcane juice, strawberry juice, watermelon juice, passion fruit juice, celery juice, carrot juice, potato juice, beet juice, parsley juice, tomato juice, watercress juice and turnip juice.

The following specific examples are included as illustrative of the compositions and methods described herein. These examples are in no way intended to limit the scope of the disclosure.

### EXAMPLES

### Example 1: Pea Protein Solutions (Formulations 1-4)

Pea protein solutions were prepared using pea protein isolate in powder form (NOW Foods) as the protein, with and without gellan gum (Ticagel^{®} Gellan HS NGMO, a high acyl gellan gum from TIC Gums) as the stabilizer, and with and without PGA 500 (Amano Enzyme Inc.) as the protein deamidating enzyme, as indicated in the table below.

For example, a 10% (w/v) solution of pea protein in water was prepared and mixed with a 0.2% (w/v) solution of gellan gum in water, to arrive at an aqueous solution with 3% (w/v) pea protein and 0.03-0.05% (w/v) gellan gum. For the PGA 500-containing solutions, the enzyme was added at an amount of 2% (w/w) of the protein, and incubated at 50°C for 3 hours. For the formulations that did not contain PGA 500, the solutions were heated to 50°C without incubation. The solution was acidified to a pH of 4.0-4.5 with citric acid, and then subjected to heat treatment at 85°C for 10 minutes. The other formulations were made by a similar process.

The resultant solutions were stored at 4°C in a laboratory VWR refrigerator and evaluated after 24 hours and 72 hours by (i) visual inspection, (2) measuring soluble protein content of the supernatant at absorbance at 280 nm, (3) measuring viscosity with a viscometer (AMETEK BROOKFIELD), and (4) measuring pH. The results are reported in the table below.

| **Formulation** | **1** | **2** | **3** | **4** |
|---|---|---|---|---|
| Protein glutaminase deamidating enzyme | - | + | + | - |
| Gellan gum | - | + | - | + |
| Visual | Solution separated | Solution dispersed | Solution separated | Solution separated |
| Absorbance 280 nm | 1.0 | 27.6 | 0.9 | 0.9 |
| Viscosity (mPa·s) | 2.1 | 25.2 | 1.8 | 3.3 |
| pH | 4.5 | 4.5 | 4.6 | 4.4 |

The results show that at a pH of about 4.5, the formulation according to the present disclosure (comprising PGA 500 and gum) (Formulation 2) was stable against precipitation of the protein, as indicated by a dispersed versus separated appearance and higher absorbance (reflecting more solubilized protein).

### Example 2: Soy Protein Solutions (Formulations 5-12)

Soy protein solutions were prepared using soy protein isolate in powder form (NOW Foods) as the protein, with and without gellan gum (Ticagel^{®} Gellan HS NGMO from TIC Gums) as the stabilizer, and with and without PGA 500 (Amano Enzyme Inc.) as the protein deamidating enzyme, as indicated in the table below.

For example, a 10% (w/v) solution of soy protein in water was prepared and mixed with a 0.2% (w/v) solution of gellan gum in water, to arrive at an aqueous solution with 3% (w/v) soy protein and 0.10% (w/v) gellan gum. For the PGA 500-containing formulations, the enzyme was added at an amount of 2% (w/w) of the protein, and incubated at 50°C for 3 hours. For the formulations that did not contain PGA 500, the solutions were heated to 50°C without incubation. The solution was acidified to a pH of 4.0-4.6 with citric acid, and then subjected to heat treatment at 85°C for 10 minutes. The other formulations were made by a similar process.

The resultant solutions were stored at 4°C in a laboratory VWR refrigerator and evaluated as described for Example 1 above. The results are reported in the table below.

| **Formulation** | **5** | **6** | **7** | **8** | **9** | **10** | **11** | **12** |
|---|---|---|---|---|---|---|---|---|
| Protein glutaminase deamidating enzyme | - | + | + | - | - | + | + | - |
| Gellan gum | - | + | - | + | - | + | - | + |
| Visual | Solution separated | Solution dispersed | Solution separated | Sediment | Solution separated | Solution dispersed | Solution separated | Solution separated |
| Absorbance 280 nm | 3.3 | 25.4 | 3.7 | 4.2 | 3.1 | 40.4 | 3.6 | 2.6 |
| Viscosity (mPa. s) | 2.1 | 39.6 | 2.7 | 1.4 | 1.8 | 191.1 | 2.7 | 44.4 |
| pH | 4.5 | 4.6 | 4.5 | 4.4 | 3.9 | 4.1 | 4.1 | 4.0 |

The results show that at acidic pH (from about 4.0 to 4.5), the formulations according to the present disclosure (comprising PGA 500 and gum) (Formulations 6 and 10 ) were stable against precipitation of the protein, as indicated by a dispersed versus separated appearance and higher absorbance.

### Example 3: Peanut Protein Solutions (Formulations 13-16)

Peanut protein solutions were prepared using peanut protein powder (Tru-Nut Company) as the protein, with and without gellan gum (Ticagel^{®} Gellan HS NGMO from TIC Gums) as the stabilizer, and with and without PGA 500 (Amano Enzyme Inc.) as the protein deamidating enzyme, as indicated in the table below.

For example, a 10% (w/v) solution of peanut protein in water was prepared and mixed with a 0.2% (w/v) solution of gellan gum in water, to arrive at an aqueous solution with 3% (w/v) peanut protein and 0.02% (w/v) gellan gum. For the PGA 500-containing formulations, the enzyme was added at an amount of 2% (w/w) of the protein, and incubated at 50°C for 3 hours. For the formulations that did not contain PGA 500, the solutions were heated to 50°C without incubation. The solution was acidified to a pH of 4.0-4.5 with citric acid, and then subjected to heat treatment at 85°C for 10 minutes. The other formulations were made by a similar process.

The resultant solutions were stored at 4°C in a laboratory VWR refrigerator and evaluated as described for Example 1 above. The results are reported in the table below.

| **Formulation** | **13** | **14** | **15** | **16** |
|---|---|---|---|---|
| Protein glutaminase deamidating enzyme | - | + | + | - |
| Gellan gum | - | + | - | + |
| Visual | Solution separated | Minor Sediment | Solution separated | Solution separated |
| Absorbance 280 nm | 5.8 | 10.7 | 6.0 | 6.4 |
| Viscosity (mPa·s) | 1.5 | 12.6 | 3.6 | 4.8 |
| pH | 4.4 | 4.2 | 4.6 | 4.4 |

The results show that at acidic pH (about 4.0), the formulation according to the present disclosure (comprising PGA 500 and gum) (Formulation 14) was more stable against precipitation of the protein, as indicated by higher absorbance (reflecting more solubilized protein) as compared to the other formulations. The observed minor sediment may be due to the nature of the peanut protein and the fact that the treatment conditions were not optimized for peanut protein.

### Example 4: Cricket Protein Solutions (Formulations 17-24)

Cricket protein solutions were prepared using cricket flour containing 68% (w/w) of cricket protein (LITHIC), with and without gellan gum (Ticagel^{®} Gellan HS NGMO from TIC Gums) as the stabilizer, and with and without PGA 500 (Amano Enzyme Inc.) as the protein deamidating enzyme, as indicated in the table below.

For example, a 10% (w/v) solution of cricket flour in water was prepared and mixed with a 0.2% (w/v) solution of gellan gum in water, to arrive at an aqueous solution with 3% (w/v) cricket flour and .03% (w/v) gellan gum. For the PGA 500-containing solutions, the enzyme was added at an amount of 2% (w/w) of the protein, and incubated at 50°C for 3 hours. For the formulations that did not contain PGA 500, the solutions were heated to 50°C without incubation. The solution was acidified to a pH of 4.0-4.5 with citric acid, and then subjected to heat treatment at 85°C for 10 minutes. The other formulations were made by a similar process.

The resultant solutions were stored at 4°C in a laboratory VWR refrigerator and evaluated as described for Example 1 above. The results are reported in the table below.

| **Formulation** | **17** | **18** | **19** | **20** | **21** | **22** | **23** | **24** |
|---|---|---|---|---|---|---|---|---|
| Protein glutaminase deamidating enzyme | - | + | + | - | - | + | + | - |
| Gellan gum | - | + | - | + | - | + | - | + |
| Visual | Solution separated | Solution dispersed | Solution separated | Sediment | Solution separated | Solution dispersed | Solution separated | Solution separated |
| Absorbance 280 | 17.1 | 40.5 | 16.5 | 32.4 | 17.4 | 29.9 | 16.1 | 17.1 |
| Viscosity (mPa. s) | 1.8 | 10.8 | 1.2 | 2.1 | 1.4 | 2.1 | 1.5 | 2.2 |
| pH | 4.3 | 4.5 | 4.3 | 4.5 | 4.1 | 4.0 | 3.9 | 4.0 |

The results show that at acidic pH (about 4.0 to about 4.5), the formulations according to the present disclosure (comprising PGA 500 and gum) (Formulations 18 and 22) were more stable against precipitation of the protein, as indicated by higher absorbance values (reflecting more solubilized protein) as compared to the other formulations. As Formulations 17 to 20 are formulated around pH 4.5 (with some measurement error) while Formulations 21 to 24 are around pH 4.0, the lower pH in Formulation 22 (pH 4.0) versus Formulation 18 (pH 4.5) explains the lower absorbance and viscosity observed with Formulation 22, as less protein is in suspension due to lower pH.

### Example 5: Hemp Protein Solution (Formulations 25-28)

Hemp protein solutions were prepared using hemp protein powder (Nutiva) as the protein, with and without carrageenan gum (Ticaloid^{®} 750 from TIC Gums) as the stabilizer, and with and without PGA 500 (Amano Enzyme Inc.) as the protein deamidating enzyme, as indicated in the table below.

For example, a 10% (w/v) solution of hemp protein in water was prepared and mixed with a 1.0% (w/v) solution of carrageenan gum in water, to arrive at an aqueous solution with 3% (w/v) hemp protein and 0.5% (w/v) carrageenan gum. For the PGA 500-containing solutions, the enzyme was added at an amount of 2% (w/w) of the protein, and incubated at 50°C for 3 hours. For the formulations that did not contain PGA 500, the solutions were heated to 50°C without incubation. The solution was acidified to a pH of 4.0-4.5 with citric acid, and then subjected to heat treatment at 85°C for 10 minutes. The other formulations were made by a similar process.

The resultant solutions were stored at 4°C in a laboratory VWR refrigerator and evaluated as described for Example 1 above. The results are reported in the table below.

| **Formulation** | **25** | **26** | **27** | **28** |
|---|---|---|---|---|
| Protein glutaminase deamidating enzyme | - | + | + | - |
| Carrageenan gum | - | + | - | + |
| Visual | Solution separated | Solution dispersed | Solution separated | Sediment |
| Absorbance 280 | 4.2 | 9.3 | 3.5 | 13.2 |
| Viscosity (mPa·s) | 1.6 | 132.0 | 4.8 | 42.9 |
| pH | 4.7 | 4.6 | 4.6 | 4.8 |

The results show that at acidic pH (about 4.5), the formulation according to the present disclosure (comprising PGA 500 and gum) (Formulation 26) was stable against precipitation of the protein, as indicated by a dispersed versus separated appearance and higher absorbance value (reflecting more solubilized protein).

### Example 6: Pea Protein Solutions with pH from 3.5 to 7.0 (Formulations 29-37)

Pea protein solutions were prepared using pea protein isolate in powder form (NOW Foods) as the protein, with and without gum as the stabilizer, and with and without PGA 500 (Amano Enzyme Inc.) as the protein deamidating enzyme, as indicated in the tables below. The following gums from TIC Gums were used: gellan (Ticagel^{®} Gellan HS NGMO), pectin (Pre-Hydrated^{®} Pectin 1694 Powder, carboxymethyl cellulose (CMC, Pre-Hydrated^{®} Ticalose^{®} CMC 2500 Powder), alginate (TICA-algin^{®} HG-400 Powder) and tara gum (TIC Pretested^{®} Tara Gum 100).

For example, a 10% (w/v) solution of pea protein in water was prepared and mixed with a gum solution in water, to arrive at an aqueous solution with 3% (w/v) pea protein and the concentration of gum (% w/v) shown in the tables below. For the PGA 500-containing formulations (the "A" formulations in the tables below), the enzyme was added at an amount of 2% (w/w) of the protein and incubated at 50°C for 3 hours. For the formulations that did not contain PGA 500 (the "B" formulations in the tables below), the solutions were heated to 50°C without incubation. The solution was acidified with citric acid to a pH ranging from 3.5 to 7 (as shown in the tables), and then subjected to heat treatment at 85°C for 10 minutes. The other formulations were made by a similar process.

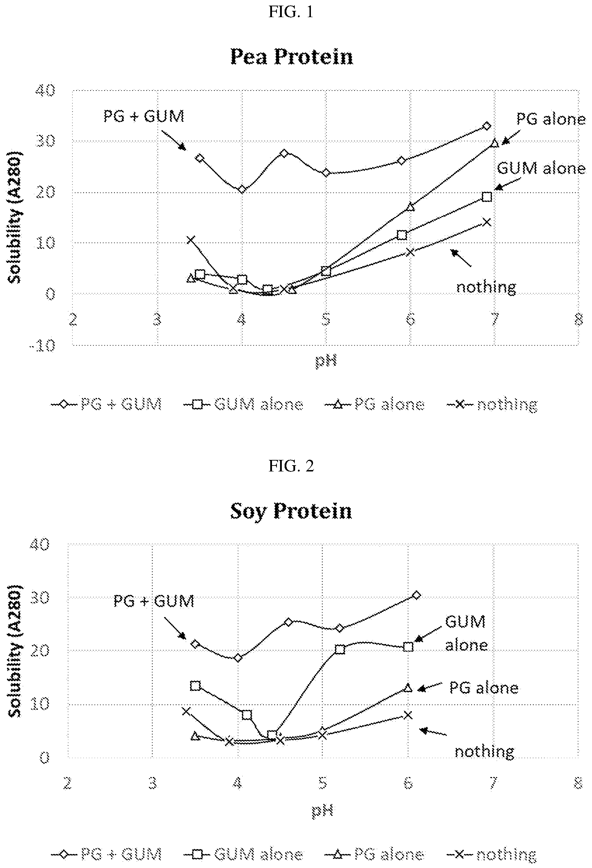

The resultant solutions were stored at 4°C in a laboratory VWR refrigerator, initially evaluated after 24 hours and then evaluated at regular intervals for up to two months (i) by visual inspection, (ii) by measuring soluble protein content of the supernatant at absorbance at 280 nm, and (iii) by measuring pH. The results are reported in the tables below and are shown in FIG. 1.

| **Form.** | **29A** | **30A** | **31A** | **32A** | **33A** | **34A** | **35A** | **36A** | **37A** |
|---|---|---|---|---|---|---|---|---|---|
| Enzyme* | + | + | + | + | + | + | + | + | + |
| Gum | Gellan 0.01% | Gellan 0.01% | Gellan 0.015% | Gellan 0.03% | Gellan 0.05% | Gellan 0.05% | Gellan 0.05% | Pectin 0.6% | Pectin 0.6% |
| | | | | | | | | | CMC 0.5% |
| | | | | | Pectin/ 0.62% | CMC 0.07% | Alginate 0.062% | CMC 0.4% | |
| | | | | | | | | | Tara 0.1% |
| Visual | Solution dispersed | Solution dispersed | Solution dispersed | Solution dispersed | Solution dispersed | Solution dispersed | Solution dispersed | Solution dispersed | Solution dispersed |
| Absorb. 280 nm | 33.0 | 26.2 | 23.8 | 27.6 | 20.6 | 12.4 | 13.2 | 32.8 | 28.9 |
| pH | 6.9 | 5.9 | 5 | 4.5 | 4 | 4 | 4 | 3.5 | 3.6 |

| **Form.** | **29B** | **30B** | **31B** | **32B** | **33B** | **34B** | **35B** | **36B** | **37B** |
|---|---|---|---|---|---|---|---|---|---|
| Enzyme* | - | - | - | - | - | - | - | - | - |
| Gum | Gellan 0.01% | Gellan 0.01% | Gellan 0.015% | Gellan 0.03% | Gellan 0.05% | Gellan 0.05% | Gellan 0.05% | Pectin 0.6% | Pectin 0.6% |
| | | | | | | | | | CMC 0.5% |
| | | | | | Pectin 0.62% | CMC 0.07% | Alginate 0.062% | CMC 0.4% | |
| | | | | | | | | | Tara 0.1% |
| Visual | Sediment | Sediment | Sediment | Solution separated | Solution separated | Solution separated | Solution separated | Sediment | Sediment |
| Absorb. 280 nm | 19.2 | 11.6 | 4.6 | 0.9 | 2.9 | 0.9 | 1.2 | 15.4 | 13.1 |
| pH | 6.9 | 5.9 | 5 | 4.3 | 4 | 4 | 4 | 3.5 | 3.6 |

| | | | | | | | | | |
|---|---|---|---|---|---|---|---|---|---|
| Enzyme* = Protein glutaminase deamidating enzyme PGA 500 Enzyme* = Protein glutaminase deamidating enzyme PGA-500 | | | | | | | | | |

The results show that at a pH of from about 3.5 to about 7, the formulations according to the present disclosure (comprising PGA 500 and gum) (Formulations 29A-37A) were stable against precipitation of the protein, as indicated by a dispersed versus separated appearance and higher absorbance (reflecting more solubilized protein). On the other hand, formulations without protein deamidating enzyme were less stable at pH below about 4.0.

### Example 7: Soy Protein Solutions with pH from 3.5 to 7.0 (Formulations 38-45)

Soy protein solutions were prepared and evaluated as described in Example 6, using soy protein isolate in powder form (NOW Foods) as the protein. Results are reported in the tables below and are shown in FIG. 2.

| **Formulation** | **38A** | **39A** | **40A** | **41A** | **42A** | **43A** | **44A** | **45A** |
|---|---|---|---|---|---|---|---|---|
| Protein glutaminase deamidating enzyme | + | + | + | + | + | + | + | + |
| Gum | Gellan 0.015% | Gellan 0.02% | Gellan 0.02% | Gellan 0.02% | Gellan 0.03% | Gellan 0.05% | Gellan 0.03% | Pectin 0.6% |
| | | | | | | Pectin 0.6% | Pectin 0.6% | CMC 0.4% |
| Visual | Solution dispersed | Solution dispersed | Solution dispersed | Solution dispersed | Solution dispersed | Solution dispersed | Solution dispersed | Solution dispersed |
| Absorbance 280 | 43.6 | 47.0 | 30.5 | 24.3 | 25.4 | 21.4 | 18.7 | 21.4 |
| pH | 6.9 | 6.9 | 6.1 | 5.2 | 4.6 | 4.0 | 4.0 | 3.5 |

| **Formulation** | **38B** | **39B** | **40B** | **41B** | **42B** | **43B** | **44B** | **45B** |
|---|---|---|---|---|---|---|---|---|
| Protein glutaminase deamidating enzyme | - | - | - | - | - | - | - | - |
| Gum | Gellan 0.015% | Gellan 0.02% | Gellan 0.02% | Gellan 0.02% | Gellan 0.03% | Gellan 0.05% | Gellan 0.03% | Pectin 0.6% |
| | | | | | | Pectin/ 0.6% | Pectin 0.6% | CMC 0.4% |
| Visual | Solution dispersed | Solution dispersed | Solution dispersed | Solution dispersed | Solution separated | Solution separated | Solution separated | Sediment |
| Absorbance 280 | 30.4 | 23.9 | 20.8 | 20.3 | 4.2 | 5.3 | 8.1 | 13.5 |
| pH | 7 | 7 | 6 | 5.2 | 4.4 | 4 | 4.1 | 3.5 |

The results show that the formulations according to the present disclosure (comprising PGA 500 and gum) (Formulations 38A-45A) were stable against precipitation of the protein across a pH range from about 3.5 to about 7, as indicated by a dispersed versus separated appearance and higher absorbance (reflecting more solubilized protein). In contrast, the other formulations (comprising gum but not PGA 500) were not stable against precipitation of the protein at pH below 5.2. (*see* results reported for pH 4.4 to 3.5).

### Example 8: Hemp Protein Solutions with pH from 3.5 to 6.5 (Formulations 46-52)

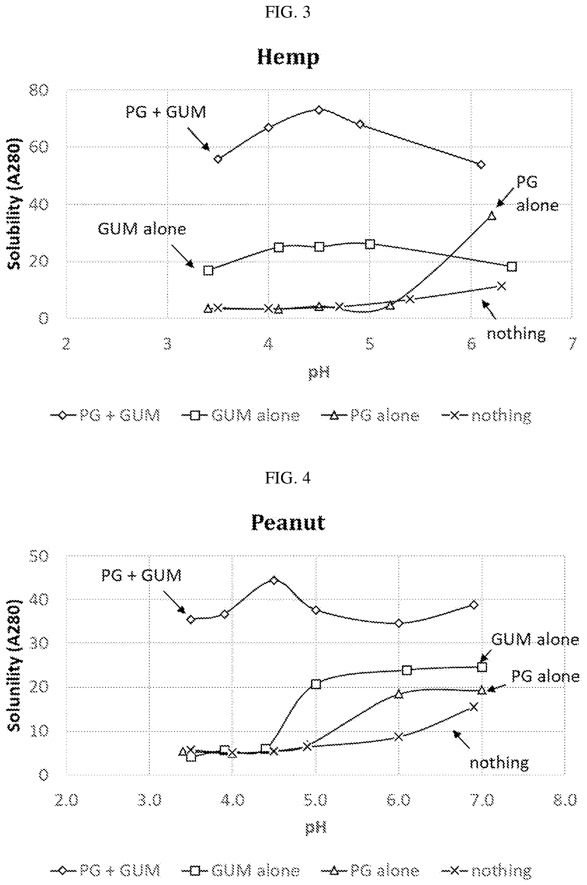

Hemp protein solutions were prepared and evaluated as described in Example 6, using hemp protein powder (Nutiva) as the protein. Results are reported in the tables below and are shown in FIG. 3.

| **Formulation** | **46A** | **47A** | **48A** | **49A** | **50A** | **51A** | **52A** |
|---|---|---|---|---|---|---|---|
| Protein glutaminase deamidating enzyme | + | + | + | + | + | + | + |
| Gum | Gellan 0.015% | Gellan 0.02% | Gellan 0.015% | Gellan 0.02% | Gellan 0.02% | Gellan 0.09% | Gellan 0.1% |
| | | CMC 0.2% | CMC 0.2% | CMC 0.2% | CMC 0.2% | | |
| Visual | Solution dispersed | Solution dispersed | Solution dispersed | Solution dispersed | Solution dispersed | Solution dispersed | Solution dispersed |
| Absorbance 280 | 53.9 | 68.0 | 73.2 | 75.0 | 66.8 | 55.7 | 53.8 |
| pH | 6.1 | 4.9 | 4.5 | 4.5 | 4.0 | 3.5 | 3.5 |

| **Formulation** | **46B** | **47B** | **48B** | **49B** | **50B** | **51B** | **52B** |
|---|---|---|---|---|---|---|---|
| Protein glutaminase deamidating enzyme | - | - | - | - | - | - | - |
| Gum | Gellan 0.015% | Gellan 0.02% | Gellan 0.015% | Gellan 0.02% | Gellan 0.02% | Gellan 0.09% | Gellan 0.1% |
| | | CMC 0.2% | CMC 0.2% | CMC 0.2% | CMC 0.2% | | |
| Visual | Sediment | Sediment | Sediment | Solution dispersed | Sediment | Solution separated | Solution separated |
| Absorbance 280 | 18.4 | 26.3 | 25.2 | 26.6 | 25.1 | 17.0 | 19.3 |
| pH | 6.4 | 5 | 4.5 | 4.4 | 4.1 | 3.4 | 3.6 |

The results show that the formulations according to the present disclosure (comprising PGA 500 and gum) (Formulations 46A-52A) were more stable against precipitation of the protein across a pH range from about 3.5 to about 7, as indicated by a dispersed appearance and higher absorbance (reflecting more solubilized protein).

### Example 9: Peanut Protein Solutions with pH from 3.5 to 7.0 (Formulations 53-59)

Peanut protein solutions were prepared and evaluated as described in Example 6, using peanut protein powder (Tru-Nut Company) as the protein and xanthan gum (Pre-Hydrated^{®} Ticaxan^{®} Xanthan EC NGMO from TIC Gums). The results are reported in the tables below and are shown in FIG. 4.

| **Formulation** | **53A** | **54A** | **55A** | **56A** | **57A** | **58A** | **59A** |
|---|---|---|---|---|---|---|---|
| Protein glutaminase deamidating enzyme | + | + | + | + | + | + | + |
| Gum | Gellan 0.02% | Gellan 0.01% | Gellan 0.018% | Gellan 0.06% | Gellan 0.04% | Xanthan 0.3% | Pectin 0.6% |
| | | CMC 0.1% | | | | CMC 0.2% | CMC 0.2% |
| Visual | Solution dispersed | Solution dispersed | Solution dispersed | Solution dispersed | Solution dispersed | Solution dispersed | Solution dispersed |
| Absorbance 280 | 38.8 | 34.6 | 37.7 | 52.5 | 44.4 | 41.5 | 35.5 |
| pH | 6.9 | 6.0 | 5.0 | 4.5 | 4.5 | 4.2 | 3.5 |

| **Formulation** | **53B** | **54B** | **55B** | **56B** | **57B** | **58B** | **59B** |
|---|---|---|---|---|---|---|---|
| Protein glutaminase deamidating enzyme | - | - | - | - | - | - | - |
| Gum | Gellan 0.02% | Gellan 0.01% | Gellan 0.018% | Gellan 0.06% | Gellan 0.04% | Xanthan 0.3% | Pectin 0.6% |
| | | CMC/ 0.1% | | | | CMC 0.2% | CMC 0.2% |
| Visual | Solution dispersed | Solution dispersed | Sediment | Solution separated | Solution separated | Solution separated | Sediment |
| Absorbance 280 | 24.6 | 23.9 | 20.7 | 6.8 | 6.0 | 5.3 | 4.2 |
| pH | 7.0 | 6.1 | 5.0 | 4.5 | 4.4 | 4.1 | 3.5 |

The results show that the formulations according to the present disclosure (comprising PGA 500 and gum) (Formulations 53A-59A) were stable against precipitation of the protein across a pH range from about 3.5 to about 7, as indicated by a dispersed appearance and higher absorbance (reflecting more solubilized protein), while the other formulations were less stable, particularly at more acidic pH values.

### Example 10: Cricket Protein Solutions with pH from 3.5 to 7.0 (Formulations 60-67)

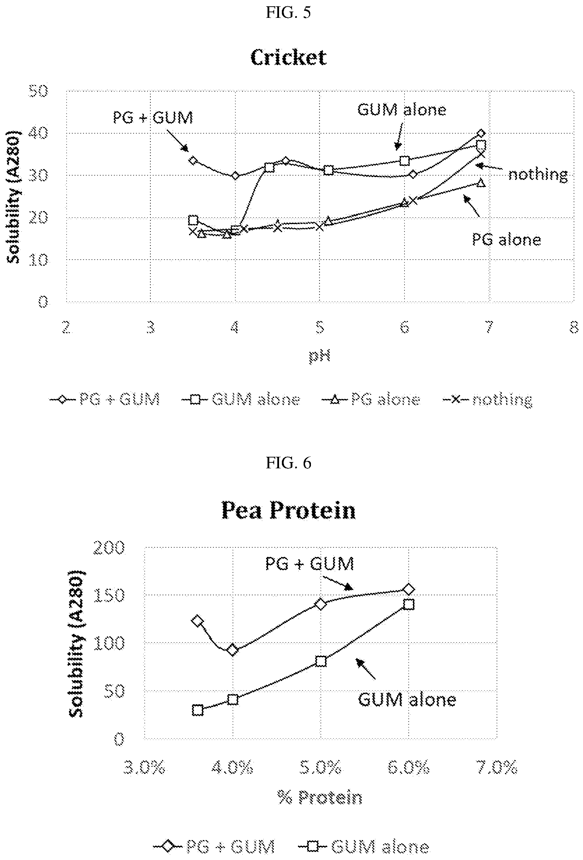

Cricket protein solutions were prepared and evaluated in the same manner as described in Example 6, using cricket flour containing 68% (w/w) of cricket protein (LITHIC). The results are reported in the tables below and are shown in FIG. 5.

| **Formulation** | **60A** | **61A** | **62A** | **63A** | **64A** | **65A** | **66A** | **67A** |
|---|---|---|---|---|---|---|---|---|
| Protein glutaminase deamidating enzyme | + | + | + | + | + | + | + | + |
| Gum | Gellan 0.02% | Gellan 0.02% | Gellan 0.0075% | Xanthan 0.15% | Gellan 0.03% | Gellan 0.07% | CMC 0.2% | Pectin/ 0.5% |
| | | | | | | | | Gellan/ 0.05% |
| Visual | Solution dispersed | Solution dispersed | Sediment | Solution dispersed | Solution dispersed | Solution dispersed | Solution dispersed | Solution dispersed |
| Absorbance 280 | 39.9 | 31.5 | 31.0 | 33.6 | 29.9 | 20.7 | 33.5 | 25.0 |
| pH | 6.9 | 6.1 | 5.1 | 4.6 | 4.0 | 3.6 | 3.5 | 3.6 |

| **Formulation** | **60B** | **61B** | **62B** | **63B** | **64B** | **65B** | **66B** | **67B** |
|---|---|---|---|---|---|---|---|---|
| Protein glutaminase deamidating enzyme | - | - | - | - | - | - | - | - |
| Gum | Gellan 0.02% | Gellan 0.02% | Gellan 0.0075% | Xanthan 0.15% | Gellan 0.03% | Gellan 0.07% | CMC 0.2% | Pectin 0.5% |
| | | | | | | | | Gellan 0.05% |
| Visual | Solution dispersed | Sediment | Sediment | Solution dispersed | Solution separated | Solution separated | Solution separated | Solution separated |
| Absorbance 280 | 37.2 | 38.7 | 31.4 | 31.9 | 17.1 | 16.4 | 19.5 | 18.2 |
| pH | 6.9 | 6.0 | 5.1 | 4.4 | 4.0 | 3.7 | 3.5 | 3.4 |

The results show that the formulations according to the present disclosure (comprising PGA 500 and gum) (Formulations 60A-67A) were stable against precipitation of the protein across a pH range from about 3.5 to about 7, as indicated by a dispersed appearance and higher absorbance (reflecting more solubilized protein). Sediment was observed with Formulation 62A, which may be attributed to the specific amount of gellan gum was not enough to completely prevent protein sedimentation used at the pH of 5.1. Most of the other formulations were less stable, particularly at more acidic pH values (e.g., below about 4.5). While not wanting to be bound by theory, it could be that the cricket protein used was not pure cricket protein, but included impurities, including non-protein impurities.

### Example 11: Pea Protein Solutions with Homogenization (Formulations 68-71)

Pea protein solutions were prepared using pea protein isolate in powder form (NOW Foods) as the protein, with and without gum (gellan: Ticagel^{®} Gellan HS NGMO; pectin: Pre-Hydrated^{®} Pectin 1694 Powder; both from TIC Gums) as the stabilizer, and with and without PGA 500 (Amano Enzyme Inc.) as the protein deamidating enzyme, as indicated in the table below.

For example, a solution was prepared by hydrating 0.075% (w/w) gellan gum and 0.45% (w/w) pectin in water. Pea protein was added to achieve pea protein solutions having different amounts of pea protein as shown in the table below. For the PGA 500-containing formulations, the enzyme was added at an amount of 0.67% ~ 1.8% (w/w) of the protein and incubated at 50°C for 3 hours. For the formulations that did not contain PGA 500, the solutions were heated to 50°C without incubation. For acidification 1M (molar) citric acid was used to attain the specified pH. The acidified solution was subjected to homogenization with 2,000 ~ 2,500 psi, and then subjected to heat treatment at 85°C for 10 minutes. The other formulations were made by a similar process.

The activity of the protein deamidating enzyme was determined in accordance to Example 16.

The resultant solutions were stored at 4°C in a laboratory VWR refrigerator and evaluated as described for Example 6 above. The results are reported in the table below and are shown in FIG.6.

| **Formulation** | **68A** | **68B** | **69A** | **69B** | **70A** | **70B** | **71A** | **71B** |
|---|---|---|---|---|---|---|---|---|
| % protein | 3.6% | 3.6% | 4.0% | 4.0% | 5.0% | 5.0% | 6.0% | 6.0% |
| Protein glutaminase deamidating enzyme (%-protein) | - | 1.8% | - | 0.67% | - | 0.67% | - | 0.67% |
| Protein glutaminase deamidating enzyme (u/300mL sol) | - | 150U | - | 60U | - | 75U | - | 90U |
| Gum | Gellan 0.05% | Gellan 0.05% | Gellan 0.05% | Gellan 0.05% | Gellan 0.05% | Gellan 0.05% | Gellan 0.05% | Gellan 0.05% |
| | Pectin 0.3% | Pectin 0.3% | Pectin 0.3% | Pectin 0.3% | Pectin 0.3% | Pectin 0.3% | Pectin 0.3% | Pectin 0.3% |
| Visual | Sediment | Solution dispersed | Sediment | Solution dispersed | Sediment | Solution dispersed | Sediment | Solution dispersed |
| Absorbance 280 | 30.6 | 123.2 | 41.2 | 92.8 | 81.2 | 141.0 | 140.4 | 156.2 |
| pH | 4.0 | 4.2 | 4.2 | 4.3 | 4.3 | 4.4 | 4.4 | 4.5 |

| | | | | | | | | |
|---|---|---|---|---|---|---|---|---|
| u/300 mL sol = units per 300 mL of solution (enzyme activity) | | | | | | | | |

The results show that at acidic pH (about 4.0 to about 4.5), the formulations according to the present disclosure (comprising PGA 500 and gum) (Formulations 68B-71B) were stable against precipitation of the protein, as indicated by a dispersed appearance and higher absorbance (reflecting more solubilized protein). These results show that the improved stability achieved with the formulas as disclosed herein is maintained even after homogenization.

### Example 12: Soy Protein Solutions with Homogenization and Juice Concentrate (Formulations 72-74)

Soy protein solutions were prepared using soy protein isolate in powder form (NOW Foods) as the protein, with and without gum (gellan: Ticagel^{®} Gellan HS NGMO; pectin: Pre-Hydrated^{®} Pectin 1694 Powder; both from TIC Gums) as the stabilizer, and with and without PGA 500 (Amano Enzyme Inc.) as the protein deamidating enzyme, as indicated in the table below.

For example, a solution was prepared by hydrating 0.06% or 0.075% (w/w) gellan gum and 0.45% (w/w) pectin in water. Soy protein isolate was added to achieve soy protein solutions having different amounts of soy protein as shown in the table below. For the PGA 500-containing formulations, enzyme was added at an amount of 0.67% ~ 2.4% (w/w) of the protein, and incubated at 50°C for 3 hours. For the formulations that did not contain PGA 500, the solutions were heated to 50°C without incubation. For acidification, berry juice concentrate (100% Juice Berry Blend concentrate at 65 Brix from Old Orchard) was added at a 1:2 w/w ratio. The acidified solution was subjected to homogenization with 2,000 ~ 2,500 psi, and then subjected to heat treatment at 85°C for 10 minutes. The other formulations were made by a similar process. The activity of the protein deamidating enzyme was determined in accordance to Example 16.

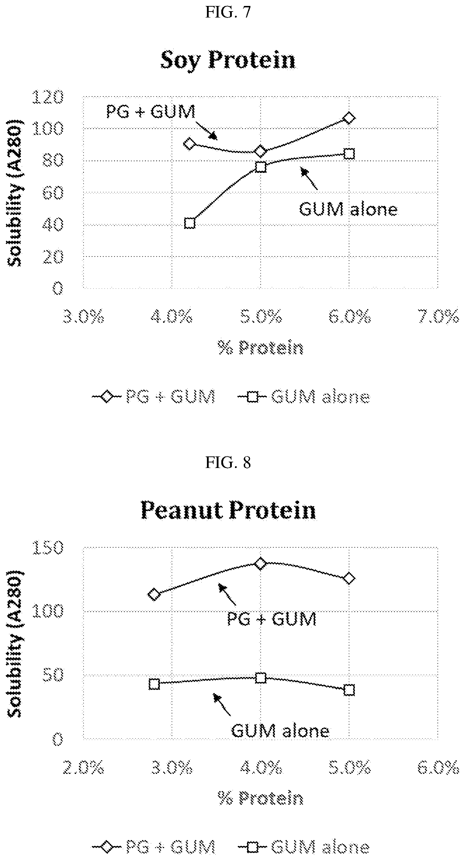

The resultant solutions were stored at 4°C in a laboratory VWR refrigerator and evaluated as described for Example 6 above. The results are reported in the table below and are shown in FIG. 7.

| **Formulation** | **72A** | **72B** | **73A** | **73B** | **74A** | **74B** |
|---|---|---|---|---|---|---|
| % protein | 4.2% | 4.2% | 5.0% | 5.0% | 6.0% | 6.0% |
| Protein glutaminase deamidating enzyme (%-protein) | - | 2.4% | - | 0.67% | - | 0.67% |
| Protein glutaminase deamidating enzyme (u/300mL sol) | - | 224U | - | 75U | - | 90U |
| Gum | Gellan 0.04% | Gellan 0.04% | Gellan 0.05% | Gellan 0.05% | Gellan 0.05% | Gellan 0.05% |
| | Pectin 0.3% | Pectin 0.3% | Pectin 0.3% | Pectin 0.3% | Pectin 0.3% | Pectin 0.3% |
| Visual | Sediment | Solution dispersed | Sediment | Solution dispersed | Sediment | Solution dispersed |
| Absorbance 280 | 41.3 | 90.4 | 76.2 | 85.8 | 84.6 | 106.8 |
| pH | 4.1 | 4.3 | 4.3 | 4.4 | 4.4 | 4.6 |

The results show that at acidic pH (about 4.0 to about 4.5), the formulations according to the present disclosure (comprising PGA 500 and gum) (Formulations 72B-74B) were stable against precipitation of the protein, as indicated by a dispersed appearance and higher absorbance (reflecting more solubilized protein). These results show that the improved stability achieved with the formulas as disclosed herein is maintained even after homogenization.

### Example 13: Peanut Protein Solutions with Homogenization and Juice Concentrate (Formulations 75-77)

Peanut protein solutions were prepared using peanut protein powder (Tru-Nut Company) as the protein, with and without gum (gellan: Ticagel^{®} Gellan HS NGMO; pectin: Pre-Hydrated^{®} Pectin 1694 Powder; both from TIC Gums) as the stabilizer, and with and without PGA 500 (Amano Enzyme Inc.) as the protein deamidating enzyme, as indicated in the table below.

For example, a solution was prepared by hydrating an amount of gellan gum and pectin as shown in the table below in water. Peanut protein was added to achieve peanut protein solutions having different amounts of peanut protein as shown in the table below. For PGA 500-containing formulations, enzyme was added at an amount of 0.67% ~ 3.6% (w/w) of the protein, and incubated at 50°C for 3 hours. For the formulations that did not contain PGA 500, the solutions were heated to 50°C without incubation For acidification, berry juice concentrate (100% Juice Berry Blend concentrate at 65 Brix from Old Orchard) was added at a 1:2 w/w ratio. The acidified solution was subjected to homogenization with 2,000 ~ 2,500 psi, and then subjected to heat treatment at 85°C for 10 minutes. The other formulations were made by a similar process. The activity of the protein deamidating enzyme was determined in accordance to Example 16.

The resultant solutions were stored at 4°C in a laboratory VWR refrigerator and evaluated as described for Example 6 above. The results are reported in the table below and are shown in FIG.8.

| **Formulation** | **75A** | **75B** | **76A** | **76B** | **77A** | **77B** |
|---|---|---|---|---|---|---|
| % protein | 2.8% | 2.8% | 4.0% | 4.0% | 5.0% | 5.0% |
| Protein glutaminase deamidating enzyme (%-protein) | - | 3.6% | - | 0.67% | - | 0.67% |
| Protein glutaminase deamidating enzyme (u/300mL sol) | - | 224U | - | 60U | - | 75U |
| Gum | Gellan 0.04% | Gellan 0.04% | Gellan 0.02% | Gellan 0.02% | Gellan 0.03% | Gellan 0.03% |
| | Pectin 0.6% | Pectin 0.6% | Pectin 0.6% | Pectin 0.6% | Pectin 0.6% | Pectin 0.6% |
| Visual | Sediment | Solution dispersed | Sediment | Solution dispersed | Sediment | Solution dispersed |
| Absorbance 280 | 43.7 | 113.4 | 48.0 | 137.4 | 38.9 | 125.6 |
| pH | 3.9 | 4.0 | 4.2 | 4.3 | 4.1 | 4.2 |

The results show that at acidic pH (about 4.0 to about 4.5), the formulations according to the present disclosure (comprising PGA 500 and gum) (Formulations 75B-77B) were stable against precipitation of the protein, as indicated by a dispersed appearance and higher absorbance (reflecting more solubilized protein). These results show that the improved stability achieved with the formulas as disclosed herein is maintained even after homogenization.

### Example 14: Almond Protein Solutions with Homogenization (Formulations 78-79)

Almond protein solutions were prepared using almond protein powder (Noosh Brands) as the protein, with and without gum as the stabilizer (gellan: Ticagel^{®} Gellan HS NGMO; pectin: Pre-Hydrated^{®} Pectin 1694 Powder; both from TIC Gums), and with and without PGA 500 (Amano Enzyme Inc.) as the protein deamidating enzyme, as indicated in the table below.

For example, a solution was prepared by hydrating 0.06% (w/w) gellan gum or 0.45% (w/w) pectin in water. Almond protein was added to achieve almond solutions having 3% (w/w) almond protein. For the PGA 500-containing formulations, enzyme was added at an amount of 3.3% (w/w) of the protein, and incubated at 50°C for 3 hours. For the formulations that did not contain PGA 500, the solutions were heated to 50°C without incubation. For acidification, berry juice concentrate (100% Juice Berry Blend concentrate at 65 Brix from Old Orchard) was added at a 1:2 w/w ratio. The acidified solution was subjected to homogenization with 2,000 ~ 2,500 psi, and then subjected to heat treatment at 85°C for 10 minutes. The activity of the protein deamidating enzyme was determined in accordance to Example 16.

The resultant solutions were stored at 4°C in a laboratory VWR refrigerator and evaluated as described for Example 6 above. The results are reported in the table below.

| **Formulation** | **78A** | **78B** | **79A** | **79B** |
|---|---|---|---|---|
| % protein | 3.0% | 3.0% | 3.0% | 3.0% |
| Protein glutaminase deamidating enzyme (%-protein) | - | 3.3% | - | 3.3% |
| Protein glutaminase deamidating enzyme (u/300mL sol) | - | 224U | - | 224U |
| Gum | Gellan 0.04% | Gellan 0.04% | Pectin 0.3% | Pectin 0.3% |
| Visual | Solution separated | Solution dispersed | Solution separated | Solution dispersed |
| Absorbance 280 | 24.4 | 132.8 | 21.5 | 244.4 |
| pH | 4.0 | 4.2 | 4.0 | 4.2 |

The results show that at acidic pH (about 4.0), the formulations according to the present disclosure (comprising PGA 500 and gum) (Formulations 78B-79B) were stable against precipitation of the protein, as indicated by a dispersed appearance and higher absorbance (reflecting more solubilized protein. These results show that the improved stability achieved with the formulas as disclosed herein is maintained even after homogenization.

The results show at a pH of from about 4.0 to about 4.2, both PGA 500 formulations with gum (Formulations 78B, and 79B) were stable as indicated by the higher absorbance values (more solubilized protein) and lack of precipitation or sediment as compared to the comparative formulations lacking PGA 500 but containing gum (Formulations 78 A and 79A), which showed separation and lower absorbance values (less solubilized proteins). These results show that the stability observed from the combination of PGA 500 and gum is maintained even with homogenization.

### Example 15: Long-Term Stability Study

A long-term stability study is being performed as follows. The formulations as described below were blended and aseptically packaged at a certified commercial pilot plant to produce commercially pasteurized and stable products, and stored for up to six months under refrigerated conditions (4°C). These products will be stable against precipitation of the protein.

| **Components** | **Control Formulation** | **Protein Deamidating Enzyme Formulation** |
|---|---|---|
| Pea protein | 4.0% | 4.0% |
| PGA-500 (Amano Enzyme Inc.) | -- | 1.0% |
| Gellan Gum (Ticagel^{®} | 0.04% | 0.04% |
| Pectin | 0.4% | 0.4% |
| Food Coloring | 1.0% | 1.0% |
| Juice Concentrate | 9.84% | 9.84% |
| Water | 84.72% | 83.72% |

For example, the following process can be used to prepare a commercially pasteurized formulation:
1. Weigh out the pectin gum, gellan gum, water, and pea protein.
2. Blend gum and water under high-shear conditions.
3. Activate gum in solution by heating to and holding at 85°C.
4. Transfer gum in water solution to 100L vat, add protein.
5. Add enzyme to vat and thoroughly mix. Transfer solution to container for incubation at 50°C for 3 hours.
6. Weigh out juice concentrate and food coloring.
7. Return solution to 100L vat and add juice concentrate and food coloring.
8. When properly mixed, feed mixture into Ultra High Temperature (UHT; *e.g.* at about 120°C for about 1 second to about 3 seconds) / High Temperature Short Time system (HTST, *e.g.,* at about 100°C for about 10 seconds to about 20 seconds) for pasteurization.
9. Feed pasteurized product into homogenizer for homogenization at 2000 PSI.
10. Bottle and cap product in a sterile environment and transfer to boxes for storage.

Stability studies to date have shown that the above-described protein deamidating enzyme formulation is stable after storage for 25 weeks at 4°C.

### Example 16: Protein Deamidating Enzyme Activity Assay

The activity of the protein deamidating enzyme may be determined by the following method, which is illustrated with reference to protein glutaminase deamidating activity. A similar assay can be carried out for protein asparaginase deamidating activity using a suitable substrate for protein asparaginase deamidation (e.g., Z-Asn-Gly).

A test solution is prepared by adding 0.1 mL of an aqueous solution containing the protein deamidating enzyme to 1 ml of 0.2 M phosphate buffer (pH 6.5) containing 30 mM Z-Gln-Gly (substrate for protein glutaminase deamidating activity assay) and is incubated for 10 minutes at 37°C. The reaction is ended by adding 1 mL of 0.4 M trichloroacetic acid (TCA) solution. A blank solution is prepared by adding 0.1 mL of an aqueous solution containing the protein deamidating enzyme to a solution containing 1 ml of 0.2M phosphate buffer (pH 6.5) containing 30 mM Z-Gln-Gly (for protein glutaminase deamidating activity assay) and 1 mL of 0.4M trichloroacetic acid (TCA) solution, and is incubated for 10 minutes at 37° C. The amount of ammonia generated by the reaction in the test solution is measured by using Ammonia Test Wako (manufactured by Wako Pure Chemical Industries, Ltd.), where ammonia concentration is determined using a calibration curve of ammonia concentration versus absorbance (at 630 nm) prepared using an ammonia standard solution (ammonium chloride). The activity of the protein deamidating enzyme may be calculated as follows (1 unit = amount of enzyme required to produce 1 µmol of ammonia per minute): Enzyme activity (U/mL) = (ammonia concentration in reaction solution (mg/L)) × (1/17.03) × (2.1/0.1) × (1/10) × Df where:
17.03 is the molecular weight of ammonia;
2.1 is the fluid volume of the enzyme reaction system (mL) in the above protocol;
0.1 is the volume of the enzyme solution (mL) in the above protocol;
10 is the reaction time (min) in the above protocol; and
Df is the dilution rate of the enzyme solution.

Collectively, these examples show that stable protein solutions can be prepared as described herein for a variety of proteins from a variety of sources, and that such protein solutions are stable against precipitation of the protein at acidic pH, including formulations acidified with fruit juice concentrate. The examples also show that protein solutions formulated as described herein are stable after homogenization.

### SEQUENCE LISTING

SEQ ID NO:1

LASVIPDVATLNSLFNQIKNQSCGTSTASSPCITFRYPVDGCYARAHKMRQILMNNGYD

CEKQFVYGNLKASTGTCCVAWSYHVAILVSYKNASGVTEKRIIDPSLFSSGPVTDTAWR

NACVNTSCGSASVSSYANTAGNVYYRSPSNSYLYDNNLINTNCVLTKFSLLSGCSPSPAP

DVSSCGF

## Claims

1. A stable protein solution, comprising:
(i) about 0.1% to about 30% w/v of protein, based on the volume of the solution;
(ii) about 0.001% to about 5% w/v of a stabilizer; based on the volume of the solution; wherein the stabilizer comprises one or more of xanthan gum, gellan gum, carrageenan gum, cassia gum, locust bean gum, tara gum, psyllium seed gum, gelatin, tamarind seed gum, gum arabic, propylene glycol alginates, pectin, galactomannan (guar gum), pullulan, carboxymethylcellulose (CMC), methylcellulose (MC), and derivatives or combinations of any thereof; and
(iii) about 0.5 U to about 50 U of protein deamidating enzyme activity or about 0.1% to about 10% w/w of a protein deamidating enzyme, based on the weight of the protein in the solution,
wherein the solution has a pH of from about 3.5 to about 7.0 and is stable against precipitation of the protein, preferably wherein the solution has a pH of from about 4.0 to about 7.0 or from about 4.0 to about 5.0.

2. The solution of claim 1, wherein the protein deamidating enzyme is
(i) a protein glutaminase deamidating enzyme that deamidates amido groups of glutamine residues of the protein, or
(ii) a protein asparaginase deamidating enzyme that deamidates amido groups of asparagine residues of the protein.

3. The solution of claim 1 or 2, wherein the protein comprises one or more selected from a plant protein, a dairy protein, and an insect protein, preferably wherein the protein comprises
(i) a plant protein selected from one or more of soy, pea, lentil, chick pea, legume, hemp, rice, nut, wheat, and gluten proteins,
(ii) whey protein, or
(iii) an insect protein selected from one or more of cricket, mole cricket, silk worm, sago worm, grasshopper, scorpion, diving beetle, waterbug, earth worm, mealworm, and spider proteins.

4. The solution of any one of the preceding claims, wherein the protein deamidating enzyme is produced by
(i) bacteria selected from *Chryseobacterium, Flavobacterium, Enpedobacter, Sphingobacterium, Aureobacterium, Myroides, Cytophagales, Actinomycetes,* and *Flavobacteriaceae,* or
(ii) a *Penicillium* microorganism, or
wherein
(iii) the protein deamidating enzyme is Protein Glutaminase Amano 500 (PGA 500).

5. The solution of any one of the preceding claims, wherein the protein deamidating enzyme comprises
(i) the amino acid sequence of SEQ ID NO:1, or a sequence having at least 75%, at least 80%, at least 85%, at least 90%, at least 95%, at least 97%, at least 98%, or at least 99% identity thereto and having protein deamidating enzyme activity, or
(ii) a variant amino acid sequence of SEQ ID NO:1, having protein deamidating enzyme activity and having one or more substitution or deletions at amino acid residues 35, 38-43, 45, 46, 49, 79-84, 103-106, 117, 142, 143, 146, 166, or 185 of SEQ ID NO:1, optionally wherein the variant sequence is at least 75%, at least 80%, at least 85%, at least 90%, at least 95%, at least 97%, at least 98%, or at least 99% identical to SEQ ID NO:1.

6. The solution of any one of claims 1-5, wherein the solution comprises:
(i) about 0.001% to about 1% w/v of a stabilizer; based on the volume of the solution; and
(ii) about 5 U to about 50 U of protein deamidating enzyme activity or about 1% to about 10% w/w of a protein deamidating enzyme, based on the weight of the protein in the solution.

7. The solution of any one of claims 1-6, wherein the solution has a viscosity of from about 10 to about 250 mPa·s, preferably wherein the solution comprises
(i) about 5% to about 15% w/v of the protein, based on the volume of the solution,
(ii) about 0.01% to about 1% w/v, or about 0.02% to about 0.5% w/v, of the stabilizer, based on the volume of the solution, or
(iii) about 5 U to about 25 U of protein deamidating activity or from about 1% w/w to about 5% w/w of the protein deamidating enzyme, based on the weight of the protein in the solution.

8. The solution of any one of the preceding claims, wherein the solution is formulated as a beverage or beverage additive for human or animal consumption.

9. A beverage or beverage additive for human or animal consumption, comprising the solution of any one of the preceding claims.

10. The beverage or beverage additive of claim 9, selected from a nutritional beverage, a sports drink, a functional protein drink, a dairy drink, a dairy smoothie, a fruit drink, a fruit smoothie, a coffee drink, a tea drink, a plant milk, a dairy creamer, and a non-dairy creamer, preferably further comprising one or more of a fruit juice, fruit juice concentrate, vegetable juice, and vegetable juice concentrate, wherein the composition preferably comprises an acidic juice or juice concentrate.

11. A method of making a solution of any one of claims 1-8 or beverage or beverage additive of claim 9 or 10, comprising:
(a) adding the protein deamidating enzyme to a solution comprising the protein and the stabilizer to obtain a mixture;
(b) incubating the mixture, preferably wherein the incubating is at a temperature of from about 40°C to about 60°C and for a period of from about 3 hours to about 24 hours with slow agitation with a pH of from about 5.0 to about 8.0; and
(c) acidifying the mixture to obtain a solution with a pH of from about 3.5 to about 7.0.

12. The method of claim 11, further comprising, prior to step (a) mixing (i) a solution comprising the protein and (ii) a solution comprising the stabilizer.

13. The method of claim 11 or 12, wherein the incubating is conducted until the enzyme reaction reaches a desired level of completion, as determined by the concentration of free ammonium ions in the solution.

14. The method of any one of claims 11 to 13, wherein the acidifying comprises adding an acidic juice or juice concentrate.

15. The method of any one of claims 11 to 14, wherein the protein deamidating enzyme is Protein Glutaminase Amano 500 (PGA 500) and/or has the amino acid sequence of SEQ ID NO:1, and the incubating is at 50°C for 3 hours.

16. The method of any one claims 11 to 15, further comprising
(i) subjecting the solution pasteurizing to a heat treatment of about 85°C for about 10 minutes, and/or
(ii) subjecting the solution to one or more treatments selected from homogenization, pasteurization, and sterilization, preferably wherein the solution is subjected to
(a) homogenization at a pressure of from about 2,000 psi to about 20,000 psi,
(b) pasteurization performed using High Temperature Short Time (HTST) pasteurization at about 100°C for about 10 seconds to about 20 seconds, Ultra High Temperature (UHT) pasteurization at about 120°C for about 1 second to about 3 seconds, or Low Temperature Long Time (LTLT) pasteurization at from about 75°C to about 85°C for about 10 minutes to about 20 minutes, or
(c) high pressure (hyperbaric) sterilization.

## Patentansprüche

1. Stabile Proteinlösung, umfassend:
(i) etwa 0,1 % bis etwa 30 % (Gewicht/Volumen) an Protein, bezogen auf das Volumen der Lösung;
(ii) etwa 0.001% bis etwa 5% (Gewicht/Volumen) eines Stabilisators, bezogen auf das Volumen der Lösung; wobei der Stabilisator eines oder mehrere von Xanthangummi, Gellangummi, Carrageenangummi, Cassiagummi, Johannisbrotkerngummi, Taragummi, Flohsamengummi, Gelatine, Tamarindensamengummi, Gummi arabicum, Propylenglycolalginaten, Pektin, Galactomannan (Guargummi), Pullulan, Carboxymethylcellulose (CMC), Methylcellulose (MC) und Derivaten oder Kombinationen davon umfasst; und
(iii) etwa 0,5 U bis etwa 50 U Protein-deamidierende Enzymaktivität oder etwa 0,1 % bis etwa 10 % (Gewicht/Gewicht) eines Protein-deamidierenden Enzyms, bezogen auf das Gewicht des Proteins in der Lösung,
wobei die Lösung einen pH-Wert von etwa 3,5 bis etwa 7,0 aufweist und stabil gegen Ausfällung des Proteins ist, vorzugsweise wobei die Lösung einen pH-Wert von etwa 4,0 bis etwa 7,0 oder von etwa 4,0 bis etwa 5,0 aufweist.

2. Lösung nach Anspruch 1, wobei das Protein-deamidierende Enzym
(i) ein Protein-Glutaminase-deamidierendes Enzym, das Amidogruppen von Glutaminresten des Proteins deamidiert, oder
(ii) ein Protein-Asparaginase-deamidierendes Enzym, das Amidogruppen von Asparaginresten des Proteins deamidiert,
ist.

3. Lösung nach Anspruch 1 oder 2, wobei das Protein eines oder mehrere, ausgewählt aus einem Pflanzenprotein, einem Milchprotein und einem Insektenprotein, umfasst, wobei das Protein vorzugsweise umfasst
(i) ein Pflanzenprotein, ausgewählt aus einem oder mehreren von Soja-, Erbsen-, Linsen-, Kichererbsen-, Hülsenfrucht-, Hanf-, Reis-, Nuss-, Weizen- und Glutenproteinen,
(ii) Molkenprotein, oder
(iii) ein Insektenprotein, ausgewählt aus einem oder mehreren Proteinen von Grille, Maulwurfsgrille, Seidenwurm, Sago-Wurm, Heuschrecke, Skorpion, Tauchkäfer, Wasserkäfer, Regenwurm, Mehlwurm und Spinne.

4. Lösung nach einem der vorhergehenden Ansprüche, wobei das Protein-deamidierende Enzym hergestellt wird durch
(i) Bakterien, ausgewählt aus *Chryseobacterium, Flavobacterium, Enpedobacter, Sphingobacterium, Aureobacterium, Myroides, Cytophagales, Actinomycetes* und *Flavobacteriaceae,* oder
(ii) einen *Penicillium-Mikroorganismus,* oder
wobei
(iii) das Protein-deamidierende Enzym Protein Glutaminase Amano 500 (PGA 500) ist.

5. Lösung nach einem der vorhergehenden Ansprüche, wobei das Protein-deamidierende Enzym umfasst
(i) die Aminosäuresequenz von SEQ ID NO: 1 oder eine Sequenz, die mindestens 75 %, mindestens 80 %, mindestens 85 %, mindestens 90 %, mindestens 95 %, mindestens 97 %, mindestens 98 % oder mindestens 99 % Identität dazu aufweist und Protein-deamidierende Enzymaktivität aufweist, oder
(ii) eine Variante der Aminosäuresequenz von SEQ ID NO: 1, die Protein-deamidierende Enzymaktivität aufweist und eine oder mehrere Substitutionen oder Deletionen an den Aminosäureresten 35, 38-43, 45, 46, 49, 79-84, 103-106, 117, 142, 143, 146, 166 oder 185 von SEQ ID NO: 1 aufweist, wobei die Sequenzvariante gegebenenfalls zu mindestens 75 %, mindestens 80 %, mindestens 85 %, mindestens 90 %, mindestens 95 %, mindestens 97 %, mindestens 98 % oder mindestens 99 % zu SEQ ID NO: 1 identisch ist.

6. Lösung nach einem der Ansprüche 1 bis 5, wobei die Lösung umfasst:
(i) etwa 0,001% bis etwa 1% (Gewicht/Volumen) eines Stabilisators, bezogen auf das Volumen der Lösung; und
(ii) etwa 5 U bis etwa 50 U Protein-deamidierende Enzymaktivität oder etwa 1 % bis etwa 10 % (Gewicht/Gewicht) eines Protein-deamidierenden Enzyms, bezogen auf das Gewicht des Proteins in der Lösung.

7. Lösung nach einem der Ansprüche 1 bis 6, wobei die Lösung eine Viskosität von etwa 10 bis etwa 250 mPa·s aufweist, wobei die Lösung vorzugsweise umfasst
(i) etwa 5 % bis etwa 15 % (Gewicht/Volumen) des Proteins, bezogen auf das Volumen der Lösung,
(ii) etwa 0,01 % bis etwa 1 % (Gewicht/Volumen) oder etwa 0,02 % bis etwa 0,5 % (Gewicht/Volumen) des Stabilisators, bezogen auf das Volumen der Lösung, oder
(iii) etwa 5 U bis etwa 25 U Protein-deamidierende Aktivität oder von etwa 1 % bis etwa 5 % (Gewicht/Gewicht) des Protein-deamidierenden Enzyms, bezogen auf das Gewicht des Proteins in der Lösung.

8. Lösung nach einem der vorhergehenden Ansprüche, wobei die Lösung als Getränk oder Getränkezusatz für den menschlichen oder tierischen Verzehr formuliert ist.

9. Getränk oder Getränkezusatz für den menschlichen oder tierischen Verzehr, umfassend die Lösung nach einem der vorhergehenden Ansprüche.

10. Getränk oder Getränkezusatz nach Anspruch 9, ausgewählt aus einem Ernährungsgetränk, einem Sportgetränk, einem funktionellen Proteingetränk, einem Milchgetränk, einem Milch-Smoothie, einem Fruchtgetränk, einem Frucht-Smoothie, einem Kaffeegetränk, einem Teegetränk, einer Pflanzenmilch, einem Milch-Creamer und einem Nicht-Milch-Creamer, vorzugsweise weiter umfassend eines oder mehrere von einem Fruchtsaft, einem Fruchtsaftkonzentrat, einem Gemüsesaft und einem Gemüsesaftkonzentrat, wobei die Zusammensetzung vorzugsweise einen sauren Saft oder ein saures Saftkonzentrat umfasst.

11. Verfahren zur Herstellung einer Lösung nach einem der Ansprüche 1 bis 8 oder eines Getränks oder eines Getränkezusatzes nach Anspruch 9 oder 10, umfassend:
(a) Zugeben des Protein-deamidierenden Enzyms zu einer Lösung, umfassend das Protein und den Stabilisator, um eine Mischung zu erhalten;
(b) Inkubieren der Mischung, wobei das Inkubieren vorzugsweise bei einer Temperatur von etwa 40°C bis etwa 60°C und für einen Zeitraum von etwa 3 Stunden bis etwa 24 Stunden unter langsamer Bewegung bei einem pH-Wert von etwa 5,0 bis etwa 8,0 erfolgt; und
(c) Ansäuern der Mischung, um eine Lösung mit einem pH-Wert von etwa 3,5 bis etwa 7,0 zu erhalten.

12. Verfahren nach Anspruch 11, weiter umfassend vor Schritt (a) das Mischen (i) einer Lösung, die das Protein umfasst, und (ii) einer Lösung, die den Stabilisator umfasst.

13. Verfahren nach Anspruch 11 oder 12, wobei das Inkubieren durchgeführt wird, bis die Enzymreaktion einen gewünschten Grad der Vollendung erreicht, wie durch die Konzentration freier Ammoniumionen in der Lösung bestimmt.

14. Verfahren nach einem der Ansprüche 11 bis 13, wobei das Ansäuern die Zugabe eines sauren Saftes oder Saftkonzentrats umfasst.

15. Verfahren nach einem der Ansprüche 11 bis 14, wobei das Protein-deamidierende Enzym Protein Glutaminase Amano 500 (PGA 500) ist und/oder die Aminosäuresequenz von SEQ ID NO: 1 aufweist und die Inkubation bei 50°C für 3 Stunden erfolgt.

16. Verfahren nach einem der Ansprüche 11 bis 15, weiter umfassend
(i) Unterziehen der pasteurisierenden Lösung einer Wärmebehandlung von etwa 85°C für etwa 10 Minuten, und/oder
(ii) Unterziehen der Lösung einer oder mehrerer Behandlungen, ausgewählt aus Homogenisierung, Pasteurisierung und Sterilisierung, wobei die Lösung vorzugsweise unterzogen wird
(a) Homogenisierung bei einem Druck von etwa 2.000 psi bis etwa 20.000 psi,
(b) Pasteurisierung durch Hochtemperatur-Kurzzeit-Pasteurisierung (HTST) bei etwa 100°C für etwa 10 Sekunden bis etwa 20 Sekunden, Ultrahochtemperatur-Pasteurisierung (UHT) bei etwa 120°C für etwa 1 Sekunde bis etwa 3 Sekunden oder Niedertemperatur-Langzeit-Pasteurisierung (LTLT) bei etwa 75°C bis etwa 85°C für etwa 10 Minuten bis etwa 20 Minuten oder
(c) (hyperbare) Hochdruck-Sterilisierung.

## Revendications

1. Solution de protéine stable, comprenant :
(i) environ 0,1 % à environ 30 % p/v de protéine, basés sur le volume de la solution ;
(ii) environ 0,001 % à environ 5 % p/v d'un stabilisant ; basés sur le volume de la solution ; dans laquelle le stabilisant comprend un ou plusieurs éléments parmi la gomme de xanthane, la gomme gellane, la gomme de carraghénine, la gomme de casse, la gomme de caroube, la gomme de tara, la gomme de graine de psyllium, la gélatine, la gomme de graine de tamarin, la gomme arabique, des alginates de propylène glycol, la pectine, le galactomannane (gomme de guar), le pullulane, la carboxyméthylcellulose (CMC), la méthylcellulose (MC) et des dérivés ou combinaisons de l'un quelconque de ceux-ci ; et
(iii) environ 0,5 U à environ 50 U d'activité d'enzyme de désamidation protéique ou environ 0,1 % à environ 10 % p/p d'une enzyme de désamidation protéique, basés sur le poids de la protéine dans la solution,
dans laquelle la solution a un pH allant d'environ 3,5 à environ 7,0 et est stable vis-à-vis de la précipitation de la protéine, de préférence dans laquelle la solution a un pH allant d'environ 4,0 à environ 7,0 ou d'environ 4,0 à environ 5,0.

2. Solution selon la revendication 1, dans laquelle l'enzyme de désamidation protéique est
(i) une enzyme de désamidation de glutaminase protéique qui désamide des groupes amido de résidus de glutamine de la protéine, ou
(ii) une enzyme de désamidation d'asparaginase protéique qui désamide des groupes amido de résidus d'asparagine de la protéine.

3. Solution selon la revendication 1 ou 2, dans laquelle la protéine comprend un ou plusieurs éléments sélectionnés parmi une protéine végétale, une protéine laitière et une protéine d'insecte, de préférence dans laquelle la protéine comprend
(i) une protéine végétale sélectionnée parmi un ou plusieurs éléments parmi des protéines de soja, pois, lentille, pois chiche, légumineuses, chanvre, riz, noix, blé et gluten,
(ii) une protéine de lactosérum, ou
(iii) une protéine d'insecte sélectionnée parmi un ou plusieurs éléments parmi des protéines de cricket, courtilière, ver à soie, ver de sagou, sauterelle, scorpion, scarabée d'eau, bélostome, ver de terre, ver de farine et araignée.

4. Solution selon l'une quelconque des revendications précédentes, dans laquelle l'enzyme de désamidation protéique est produite par
(i) des bactéries sélectionnées parmi *Chryseobacterium, Flavobacterium, Enpedobacter, Sphingobacterium, Aureobacterium, Myroides, Cytophagales, Actinomycetes* et *Flavobacteriaceae,* ou
(ii) un microorganisme de *Penicillium,* ou
dans laquelle
(iii) l'enzyme de désamidation protéique est Protein Glutaminase Amano 500 (PGA 500).

5. Solution selon l'une quelconque des revendications précédentes, dans laquelle l'enzyme de désamidation protéique comprend
(i) la séquence d'acides aminés de SEQ ID N° : 1, ou une séquence ayant au moins 75 %, au moins 80 %, au moins 85 %, au moins 90 %, au moins 95 %, au moins 97 %, au moins 98 % ou au moins 99 % d'identité à celle-ci et ayant une activité d'enzyme de désamidation protéique, ou
(ii) une séquence d'acides aminés variante de SEQ ID N° : 1, ayant une activité d'enzyme de désamidation protéique et ayant une ou plusieurs substitutions ou délétions au niveau des résidus d'acide aminé 35, 38 à 43, 45, 46, 49, 79 à 84, 103 à 106, 117, 142, 143, 146, 166 ou 185 de SEQ ID N° : 1, en option dans laquelle la séquence variante est au moins 75 %, au moins 80 %, au moins 85 %, au moins 90 %, au moins 95 %, au moins 97 %, au moins 98 % ou au moins 99 % identique à SEQ ID N° : 1.

6. Solution selon l'une quelconque des revendications 1 à 5, dans laquelle la solution comprend :
(i) environ 0,001 à environ 1 % p/v d'un stabilisant ; basé sur le volume de la solution ; et
(ii) environ 5 U à environ 50 U d'activité d'enzyme de désamidation protéique ou environ 1 % à environ 10 % p/p d'une enzyme de désamidation protéique, basés sur le poids de la protéine dans la solution.

7. Solution selon l'une quelconque des revendications 1 à 6, dans laquelle la solution a une viscosité allant d'environ 10 à environ 250 mPa·s, de préférence dans laquelle la solution comprend
(i) environ 5 % à environ 15 % p/v de la protéine, basés sur le volume de la solution,
(ii) environ 0,01 % à environ 1 % p/v, ou environ 0,02 % à environ 0,5 % p/v, du stabilisant, basés sur le volume de la solution, ou
(iii) environ 5 U à environ 25 U d'activité d'enzyme de désamidation protéique ou d'environ 1 % p/p à environ 5 % p/p de l'enzyme de désamidation protéique, basés sur le poids de la protéine dans la solution.

8. Solution selon l'une quelconque des revendications précédentes, dans laquelle la solution est formulée en tant que boisson ou additif pour boisson pour la consommation humaine ou animale.

9. Boisson ou additif pour boisson pour la consommation humaine ou animale, comprenant la solution selon l'une quelconque des revendications précédentes.

10. Boisson ou additif pour boisson selon la revendication 9, sélectionné(e) parmi une boisson nutritionnelle, une boisson pour le sport, une boisson protéique fonctionnelle, une boisson lactée, un smoothie lacté, une boisson fruitée, un smoothie fruité, une boisson à base de café, une boisson à base de thé, un lait végétal, un colorant à café laitier et un colorant à café non laitier, comprenant en outre de préférence un ou plusieurs éléments parmi un jus de fruit, un concentré de jus de fruit, un jus de légume et un concentré de jus de légume, dans laquelle/lequel la composition comprend de préférence un jus ou concentré de jus acide.

11. Procédé de fabrication d'une solution selon l'une quelconque des revendications 1 à 8 ou d'une boisson ou d'un additif pour boisson selon la revendication 9 ou 10, comprenant :
(a) ajouter l'enzyme de désamidation protéique à une solution comprenant la protéine et le stabilisant pour obtenir un mélange ;
(b) faire incuber le mélange, de préférence dans lequel l'incubation est à une température allant d'environ 40 °C à environ 60 °C et pendant une période allant d'environ 3 heures à environ 24 heures avec agitation lente avec un pH allant d'environ 5,0 à environ 8,0 ; et
(c) acidifier le mélange pour obtenir une solution avec un pH allant d'environ 3,5 à environ 7,0.

12. Procédé selon la revendication 11, comprenant en outre, avant l'étape (a), mélanger (i) une solution comprenant la protéine et (ii) une solution comprenant le stabilisant.

13. Procédé selon la revendication 11 ou 12, dans lequel l'incubation est réalisée jusqu'à ce que la réaction enzymatique atteigne un niveau d'achèvement souhaité, tel que déterminé par la concentration en ions ammonium libres dans la solution.

14. Procédé selon l'une quelconque des revendications 11 à 13, dans lequel l'acidification comprend ajouter un jus ou concentré de jus acide.

15. Procédé selon l'une quelconque des revendications 11 à 14, dans lequel l'enzyme de désamidation protéique est Protein Glutaminase Amano 500 (PGA 500) et/ou a la séquence d'acides aminés de SEQ ID N° : 1, et l'incubation est à 50 °C pendant 3 heures.

16. Procédé selon l'une quelconque des revendications 11 à 15, comprenant en outre
(i) soumettre la pasteurisation de solution à un traitement thermique d'environ 85 °C pendant environ 10 minutes, et/ou
(ii) soumettre la solution à un ou plusieurs traitements sélectionnés parmi l'homogénéisation, la pasteurisation et la stérilisation, de préférence dans lequel la solution est soumise à
(a) une homogénéisation à une pression allant d'environ 2000 psi à environ 20 000 psi,
(b) une pasteurisation réalisée en utilisant une pasteurisation haute (HTST) à environ 100 °C pendant environ 10 secondes à environ 20 secondes, une pasteurisation à ultra-haute température (UHT) à environ 120 °C pendant environ 1 seconde à environ 3 secondes, ou une pasteurisation basse (LTLT) à une température allant d'environ 75 °C à environ 85 °C pendant environ 10 minutes à environ 20 minutes, ou
(c) une stérilisation haute pression (hyperbare).
